# EUROPEAN PATENT APPLICATION

(11) **EP 4 748 332 A1**
(43) Date of publication of application: **27.05.2026**
(21) Application number: 24386132.5
(22) Date of filing: 20.11.2024
(51) Int. Cl.: A61B 34/20, A61B 17/00, A61B 34/30, A61F 2/18

(54) **SURGICAL APPARATUS**

(71) Applicant: Imperial College Innovations Limited, South Kensington, London SW7 2AZ (GB)
(72) Inventor: KASSANOS, Panagiotis, London, SW72AZ (GB); REDHEAD, William, London, SW72AZ (GB); SALINAS, Daniel Bautista, London, SW72AZ (GB); SOROUR, Omar, London, SW72AZ (GB)
(74) Representative: HGF

(57) **Abstract**

Disclosed is an apparatus for controlling insertion of an implant into an electrically conductive volume in a patient using impedance measurements, a surgical instrument for implant insertion which may comprise the apparatus, and related features. The implant may, for example, be a cochlear implant or a brain implant.

## Description

This invention relates to apparatus, methods, and surgical instruments which are suitable for use in controlling insertion of an implant, in particular, apparatus for controlling insertion of an implant into an electrically conductive volume, systems, surgical instruments, surgical systems, and surgical instrument systems.

### BACKGROUND

Currently, it can be challenging to control the insertion of an implant, such as a cochlear implant, into an electrically conductive volume (e.g. a cochlea). Potential difficulties include a lack of information about the positioning of the implant during insertion, which, if there is less than ideal positioning, can cause trauma to the volume in which the implant is being inserted, and which can cause damage, sub-optimal performance of the implanted device, or need for reimplantation.

Accordingly, there remains a need to provide improvements to apparatus for insertion of implants.

### BRIEF SUMMARY OF THE DISCLOSURE

In accordance with an aspect of this disclosure, there is provided an apparatus for controlling insertion of an implant into an electrically conductive volume in a patient, the implant comprising a plurality of electrodes arranged on an electrode contact surface of the implant, the apparatus comprising a memory configured to store computer-readable instructions; and a processor configured to execute the computer-readable instructions which, when executed, cause the apparatus to: transmit, to a pulse generator, at least a first electrode operation signal configured to generate at least a first current pulse on at least a first pair of electrodes of the implant; receive a first impedance signal from a first measurement pair of electrodes, the first impedance signal indicative of a first impedance due to interaction of the at least a first current pulse with the electrically conductive volume; receive a second impedance signal from a second measurement pair of electrodes, the second impedance signal indicative of a second impedance due to interaction of the at least a first current pulse with the electrically conductive volume, wherein the first measurement pair of electrodes and the second measurement pair of electrodes are different; determine, in dependence on the first impedance signal and the second impedance signal, and on a predetermined mapping relationship, a distance between the electrode contact surface and a wall of the electrically conductive volume; and output the determined distance between the electrode contact surface and a wall of the electrically conductive volume.

The apparatus may be further configured to determine, in dependence on the first impedance signal, the second impedance signal, and the predetermined mapping relationship, a pose of a portion of the implant within the electrically conductive volume and output the determined pose.

The apparatus may be further configured to determine, in dependence on the first impedance signal, the second impedance signal, and the predetermined mapping relationship, one or more of: intravolume pressure, implant insertion speed, insertion depth, risk of trauma to the wall of the of the electrically conductive volume, a level of trauma of the wall of the electrically conductive volume, a translocation of the implant through the wall of the electrically conductive volume, and occurrence of a tip roll-over event of the implant.

The implant may be a cochlear implant insertable into a scala tympani of a cochlea; the electrically conductive volume may be a cochlea, and the distance may be between the electrode contact surface and an inner wall of the scala tympani of the cochlea. The electrode-modiolar distance may be determined from the surface of the electrode contacts to the modiolar wall of the scala tympani.

The implant may be a brain implant insertable into a portion of a brain; the electrically conductive volume may be a brain, and the distance may be between the electrode contact surface and an internal surface of the brain. That is, for surface or cortical implants, the "wall" would be the brain surface. For deep brain implants, the "wall" would be inner parts of the brain. Different tissues have different impedance values. Knowing this beforehand help us to place the implant in the desired location in the brain.

A distal electrode of the at least a first pair of electrodes may be located in proximity to a tip of the implant, wherein the tip may be inserted first into the electrically conductive volume.

The at least a first pair of electrodes may be a single pair of electrodes; and the at least a first electrode operation signal may be a single electrode operation signal.

The at least a first pair of electrodes may comprise a first pair of electrodes and a second pair of electrodes different from the first pair of electrodes; the at least a first electrode operation signal may comprise a first electrode operation signal and a second electrode operation signal; and the at least a first current pulse generated on at least a first pair of electrodes may comprise a first current pulse generated on the first pair of electrodes and a second current pulse generated on the second pair of electrodes.

The apparatus may be configured to receive the first impedance signal and the second impedance signal simultaneously.

The first impedance signal may be indicative of a tetrapolar impedance, received based on a voltage difference between the first measurement pair of electrodes, the first measurement pair of electrodes comprising: a distal first measurement electrode positioned at a distal end of the first pair of measurement electrodes, and a proximal first measurement electrode positioned at a proximal end of the first pair of measurement electrodes; so the at least a first pair of electrodes are located between the distal and proximal first measurement electrodes.

The second impedance signal may be indicative of a bipolar impedance, received based on a voltage difference between the second pair of electrodes.

The apparatus may be configured to: transmit, to the pulse generator, a first further electrode operation signal configured to generate a first further current pulse on a further pair of electrodes of the implant, wherein the further pair of electrodes comprises at least one different electrode to the at least a first pair of electrodes so the further pair of electrodes are separated by a different amount than the at least a first pair of electrodes; receive a first further impedance signal indicative of a first further impedance due to interaction of the first further current pulse with the electrically conductive volume; and determine the distance between the electrode contact surface and a wall of the electrically conductive volume further in dependence on the first further impedance signal.

The apparatus may be further configured to: transmit, to the pulse generator, a second further electrode operation signal configured to generate a second further current pulse on a second further pair of electrodes of the implant, wherein the second further pair of electrodes comprises at least one different electrode to the at least one first pair of electrodes so the second further pair of electrodes are separated by a different amount than the at least one first pair of electrodes; receive a second further impedance signal indicative of a second further impedance due to interaction of the second further current pulse with the electrically conductive volume; and determine the distance between the electrode contact surface and a wall of the electrically conductive volume further in dependence on the second further impedance signal.

The apparatus may be configured to transmit the first further electrode operation signal and the second further operation electrode signal to the same pair of electrodes, so the first further pair of electrodes is the same as the second further pair of electrodes.

The apparatus may be further configured to: transmit, to the pulse generator, a third electrode operation signal configured to generate a third current pulse on a third pair of electrodes of the implant; and receive a third impedance signal indicative of a third impedance due to interaction of the third current pulse with the electrically conductive volume, received based on a voltage difference between a third measurement pair of electrodes, the third measurement pair of electrodes comprising: a common third electrode of both the third pair of electrodes and the third measurement pair of electrodes; and a further third electrode of the electrode array, the further third electrode different from the third pair of electrodes.

The further third electrode may be: one of the at least a first measurement pair of electrodes used to measure a tetrapolar impedance or bipolar impedance; or an electrode located between the at least a first measurement pair of electrodes.

The apparatus may be further configured to: transmit, to the pulse generator, a fourth electrode operation signal configured to generate a fourth current pulse on a fourth pair of electrodes, the fourth pair of electrodes comprising a reference electrode external to the electrodes of the implant, and a fourth electrode of the electrodes of the implant; and receive a fourth impedance signal indicative of a fourth impedance due to interaction of the fourth current pulse with the electrically conductive volume, received based on a voltage difference between a fourth pair of electrodes.

The apparatus may be further configured to: transmit one or more of a plurality of first electrode operation signals each at a different frequency, and a plurality of second electrode operation signals each at a different frequency; and determine the distance between the electrode contact surface and a wall of the electrically conductive volume in dependence on one or more of: a plurality of first impedance signals respectively received due to the plurality of first electrode operation signals, and a plurality of second impedance signals respectively received due to the plurality of second electrode operation signals.

The predetermined mapping relationship may be determined using a machine learning model trained using: first impedance signal training data, second impedance signal training data, and training data distances between the electrode contact surface and a wall of the electrically conductive volume corresponding to the first and second impedance signal training data.

The apparatus may be further configured to determine the predetermined mapping relationship; wherein the apparatus is further configured to calibrate the at least a first current pulse in dependence on anatomical and/or physiological data of the patient to obtain the predetermined mapping relationship; optionally wherein the anatomical and/or physiological data is obtained preoperatively and intraoperatively in the stages before the insertion.

The apparatus may be configured to output the determined distance between the electrode contact surface and a wall of the electrically conductive volume to one or more of: an output device configured to provide an indication of the output to a user; and a surgical instrument controllable using the output to perform insertion of the implant into an electrically conductive volume in a patient.

In accordance with another aspect of this disclosure, there is provided a system for controlling insertion of an implant into an electrically conductive volume in a patient, the system comprising any apparatus disclosed herein, and the pulse generator.

The system may further comprise a front-end amplifier configured to receive the first impedance signal and the second impedance signal simultaneously.

The system may further comprise a multiplexer configured to select at least the first pair of electrodes and the second pair of electrodes from the plurality of electrodes of the implant.

In accordance with another aspect of this disclosure, there is provided a surgical instrument for implant insertion, the surgical instrument comprising: an insertion module; a linear translation portion configured to translate the insertion module along a first linear direction to change an entry position of implantation; a rotational translation portion configured to rotationally translate the insertion module in a first plane of rotation to change the entry position of implantation; a first rotatable portion configured to rotate the supported insertion module in a yaw plane to change the angular direction of implantation; a second rotatable portion configured to rotate the insertion module in a pitch plane to change the angular direction of implantation, the pitch plane perpendicular to the yaw plane; and an implant holder movement portion configured to: translate an implant holder held in the insertion module along a direction of implantation and rotate the implant holder about an implant axis of rotation along the direction of implantation.

In accordance with another aspect of this disclosure, there is provided a surgical instrument for implant insertion, the surgical instrument comprising: a positioning module; and an insertion module; the positioning module comprising: a patient-attachable portion configured to attach to a patient; and an insertion module mechanism comprising: an insertion module support configured to support the insertion module; a translation portion configured to translate the supported insertion module along a first linear direction to change an entry position of implantation; and configured to rotationally translate the supported insertion module in a first plane of rotation to change the entry position of implantation; and a rotatable portion configured to rotate the supported insertion module in a yaw plane to change the angular direction of implantation; the insertion module comprising: a rotatable portion configured to rotate the insertion module in a pitch plane to change the angular direction of implantation; the pitch plane perpendicular to the yaw plane; and an implant holder movement portion configured to translate an implant holder held in the insertion module along a second linear direction along a direction of implantation and configured to rotate the implant holder about a first axis of rotation along the direction of implantation.

The surgical instrument may further comprise an implant holder configured to releasably hold an implant for insertion into a volume The implant holder may be detachably connected to the implant holder movement portion. The surgical instrument may in some examples further comprise a tool holder configured to releasably hold a tool for performing or assisting in the performance of a surgical procedure, e.g. by positioning and/or manipulation of the tool. The tool holder may be detachably connected to a tool holder movement portion (what is also called an implant holder movement portion in the discussion herein).

The insertion module may comprise a housing configured to at least partially house the implant holder movement portion, the housing comprising a body and a plurality of arms extended from the body, the arms configured to attach to the insertion module support.

The insertion module support may comprise an annular ring configured to connect to the plurality of arms and support the insertion module.

The positioning module may be detachably connected to the insertion module, optionally, detachably connected at the points where the annular ring is connected to each of the plurality of arms.

The positioning module may further comprise an external support portion configured to attach to an external supporting element. The surgical instrument may further comprise the external supporting element, e.g. an arm to attach to a patient bed.

The implant holder may comprise an implant contact portion, the implant contact portion comprising one or more of: a deformable material configured to conform to the shape of an implant held in the implant holder; a patterned surface configured to grip an implant held in the implant holder; and a channel (which may be called a groove or carved section) configured to adapt to the shape of an implant located therein (i.e. dimensioned to hold an implant).

The implant holder may be removably connected to the implant holder movement portion to facilitate connection and detachment of the implant holder from the implant holder movement portion. The implant holder may comprise at least one engagement recess, and the implant holder movement portion may comprise at least one wing corresponding to the at least one engagement recess, the at least one wing configured to engage with the at least one engagement recess of an implant holder connected thereto to secure the implant holder to the implant holder movement portion.

The implant holder may comprise one or plural available implant holders each having a differently dimensioned channel to allow for implantation of implants of different dimensions.

In accordance with another aspect of this disclosure, there is provided a surgical instrument for surgical tool manipulation, the surgical instrument comprising: a positioning portion; a linear translation portion configured to translate the positioning module along a first linear direction to change a tool position of a tool held by the surgical instrument; a rotational translation portion configured to rotationally translate the positioning module in a first plane of rotation to change the tool position of the tool held by the surgical instrument; a first rotatable portion configured to rotate the supported positioning module in a yaw plane to change the angular position of the tool held by the surgical instrument; a second rotatable portion configured to rotate the positioning module in a pitch plane to change the angular position of the tool held by the surgical instrument, the pitch plane perpendicular to the yaw plane; and a tool holder movement portion configured to: translate a tool holder held in the positioning module along a direction of approach, and rotate the tool holder about an axis of rotation along the direction of approach.

In accordance with another aspect of this disclosure, there is provided a surgical instrument for surgical tool manipulation, the surgical instrument comprising: a positioning module; and a positioning module; the positioning module comprising: a patient-attachable portion configured to attach to a patient; and a positioning module mechanism comprising: a positioning module support configured to support the positioning module; a translation portion configured to translate the supported positioning module along a first linear direction to change a tool position of a tool held by the surgical instrument; and configured to rotationally translate the supported positioning module in a first plane of rotation to change the tool position of the tool held by the surgical instrument; and a rotatable portion configured to rotate the supported positioning module in a yaw plane to change the angular position of the tool held by the surgical instrument; the positioning module comprising: a rotatable portion configured to rotate the positioning module in a pitch plane to change the angular position of the tool held by the surgical instrument; the pitch plane perpendicular to the yaw plane; and a tool holder movement portion configured to translate a tool holder held in the positioning module along a second linear direction along a direction of approach and configured to rotate the tool holder about a first axis of rotation along the direction of approach.

In accordance with another aspect of this disclosure, there is provided a surgical system comprising: any surgical instrument disclosed herein, and a controller; the controller configured to operate the movement of one or more of the translation portion, the rotatable portion, and the implant holder movement portion of the surgical instrument by operating a respective movement mechanism of the translation portion, the rotatable portion, and the implant holder movement portion. The movement mechanism may comprise one or more of a gear, a prismatic joint, and an other mechanism to control movement.

The surgical system may further comprise any apparatus disclosed herein, or any system disclosed herein; the apparatus or system configured to output the determined distance between the electrode contact surface and a wall of the electrically conductive volume to the controller, to cause the controller to operate the movement of the one or more of the translation portion, the rotatable portion, and the implant holder movement portion of the surgical instrument to perform implantation of the implant in the electrically conductive volume of the patient.

The apparatus or system of the surgical system may be configured to: determine, in dependence on the determined distance between the electrode contact surface and a wall of the electrically conductive volume, one or more operation controls to provide to the controller to cause the controller to operate the movement of the one or more of the translation portion, the rotatable portion, and the implant holder movement portion of the surgical instrument; and output the one or more operation controls to the controller.

The surgical system may further comprise a microscope configured to capture images of the location of insertion of an implant into an electrically conductive volume in a patient. The surgical system may further comprise a display screen apparatus configured to receive image signalling from the microscope and display the captured images of the location of insertion of the implant.

In accordance with another aspect of this disclosure, there is provided a surgical instrument system comprising any surgical instrument or surgical system disclosed herein, and a rig configured to support at least a part of the surgical instrument to facilitate loading and unloading of an implant in and from the implant holder.

### BRIEF DESCRIPTION OF THE DRAWINGS

Embodiments of the invention are further described hereinafter with reference to the accompanying drawings, in which:
Figure 1 shows schematically an apparatus for controlling insertion of an implant into an electrically conductive volume in a patient according to examples discloses herein;
Figure 2 shows schematically a cochlear implant as an example of an implant comprising a plurality of electrodes which may be inserted into an electrically conductive volume in a patient using examples disclosed herein;
Figure 3 shows schematically a system comprising an apparatus according to examples discloses herein;
Figure 4A shows data indicating a depth of implant insertion at which 90% sensitivity is confined for tetrapolar and a bipolar electrode combinations and Figure 4B illustrates schematically a series of electrodes and distances between respective electrode pairs;
Figures 5a) to e) shows change in tetrapolar impedance measurements with electrode-wall distance at five depths of insertion, and Figure 5f) shows the change in bipolar impedance measurement at those depths, for a plurality of combinations of electrodes of examples disclosed herein;
Figures 6a) to e) shows change in combined tetrapolar impedance measurements with electrode-wall distance at five depths of insertion, and Figure 6f) shows the change in combined bipolar impedance measurement at those depths, for a plurality of combinations of electrodes of examples disclosed herein;
Figure 7 shows a bar graph of precision and sensitivity for three sensing modalities of examples disclosed herein;
Figure 8 shows a model of electrical potential field in a cochlear and electrode array with changes in impedance with electrode rotation angles and electrical frequencies;
Figures 9A-J show an example surgical instrument and possible movement of portions of the instrument according to examples disclosed herein;
Figures 10A and 10B shows an example implant holder portion, and an example implant holder, each configured to releasably hold an implant according to examples disclosed herein;
Figure 11 shows a surgical system comprising a surgical instrument and an apparatus according to examples disclosed herein; and
Figure 12 shows a surgical instrument system comprising a surgical instrument and a rig according to examples disclosed herein.

### DETAILED DESCRIPTION

Electrode implants may be used in medicine in applications such as brain implants and cochlear implants. Cochlear implant (CI) systems are a successful solution for people suffering from sensorineural hearing loss, and can restore hearing perception in such patients with electrical stimulation through an electrode array placed in the scala tympani (i.e., the cochlea chamber where the electrode array is inserted) of the ear. The Cl electrode array may be inserted by hand using traditional surgical instrumentation, though this can be a challenging surgical procedure. Limitations that make the procedure challenging are the lack of visual access beyond the entry point and the handheld surgical instrumentation used during insertion. The electrode array placement can produce intracochlear trauma due to contact between the electrode array and the scala tympani walls, electrode array translocation into a neighbouring chamber, and variable insertion speeds. Intracochlear trauma may lead to residual hearing loss, affecting the patient's hearing outcome. Residual hearing loss is also a cause for surgeons and hospitals to only provide a cochlear implant to patients with severe-to-profound sensorineural hearing loss; in the US five of six patients that could benefit from a cochlear implant are discarded due to these risks.

Visual feedback available during the insertion is limited to electrode array marks on the implant, informing the surgeon about the insertion depth. The surgeon cannot rely on the force feedback from contact of the electrode array with the scala tympani walls since, by the time the surgeon's hand feels the force, some degree of trauma has been caused. A lack of feedback during insertion, both for a surgeon and a robotic system, often leads to causing trauma to the patient, who loses the remaining natural hearing, which leads to recommendation of a cochlear implant only to patients with zero or very minimal natural hearing (i.e., patients with severe-to-profound sensorineural hearing loss). Cochlear implant candidate patients are discarded due to these risks.

Therefore, a form of intraoperative feedback is needed for the surgeon during insertion. In addition, the manual insertion procedure does not allow a low and constant insertion speed to be achieved.

To solve these problems, there have been investigations into the use of robotic devices to insert the electrode array. While these devices can achieve low and constant insertion speed, they still lack intraoperative feedback that would allow for automation of the insertion procedure and allow for monitoring of the likelihood of adverse events to provide a safer insertion.

Therefore, an improved system and method for robotic implant insertion are needed. Examples disclosed herein provide alternative systems and methods to increase the safety of the electrode implant procedure and make cochlear implants accessible to more candidates. Examples disclosed herein allow for improved implant location and event detection accuracy using electrical impedance measurements.

Examples disclosed herein can identify the position and orientation of the cochlear implant electrode array within the cochlea during insertion. This information can be used to update the insertion parameters of a robotic implant insertion device to minimise or eliminate the damage created during insertion. Examples disclosed herein can detect the likelihood of an adverse event and provide a real-time insertion visualisation which the surgeon can use to monitor an autonomous insertion and assist during traditional cochlear implant surgeries. For example, the real-time position of the electrode array, and information on specific adverse events (e.g., tip roll-over or translocation into a neighbouring chamber) and insertion depth values may be obtained using examples disclosed herein.

The obtained data can be input into a controller to allow for the autonomous robotic insertion of cochlear implant electrode arrays, to help preserve natural hearing. The autonomous insertion methods disclosed herein allow for adaptation for different types of cochlear implant available, to help minimise adverse events.

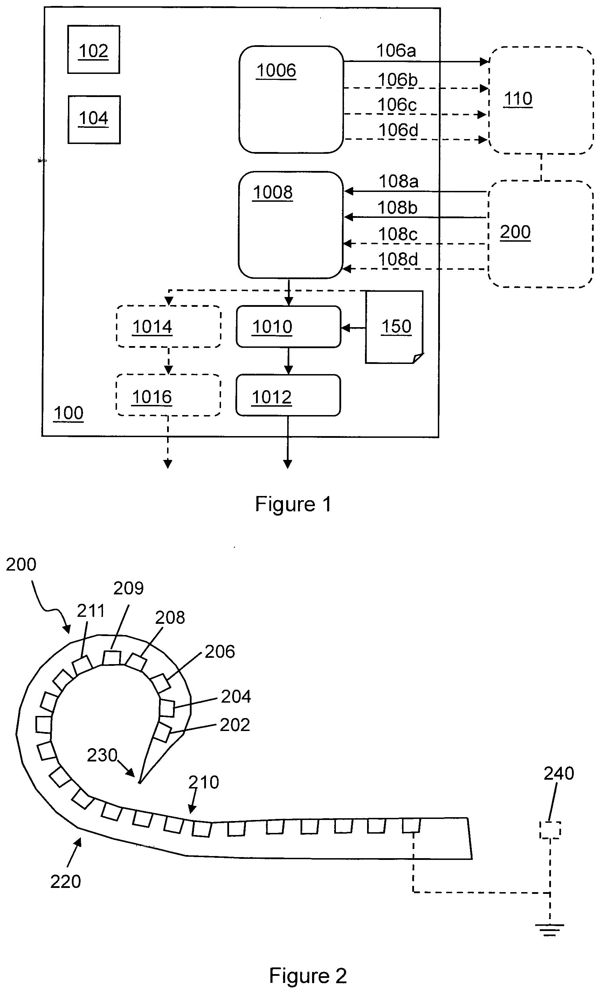

Figure 1 shows schematically an apparatus 100 for controlling insertion of an implant 200 (such as that shown in Figure 2) into an electrically conductive volume in a patient according to examples disclosed herein. The implant 200 comprises a plurality of electrodes 202, 204, 206, 208 arranged on an electrode contact surface 210 of the implant 200.

The apparatus 100 comprises a memory 102 configured to store computer-readable instructions and a processor 104 configured to execute the computer-readable instructions. When executed, the computer-readable instructions cause the apparatus 100 to: transmit 1006, to a pulse generator 110, at least a first electrode operation signal 106a configured to generate at least a first current pulse on at least a first pair of electrodes of the implant 200.

Looking to Figure 2, an example implant 200 is shown comprising a plurality of electrodes 202, 204, 206, 208 on an electrode contact surface 210. The electrodes wires connected to the exposed electrodes 202, 204, 206, 208 are housed inside a carrier material (i.e., the implant) which is made out of an insulating material. A recess is created in the outside surface of the implant to access the electrode wires. The recess is filled with conductive material (e.g. metal), and this is what is called an electrode contact, or electrode 202, 204, 206, 208. The electrode 202, 204, 206, 208 can be at the same level of the carrier material to form a contiguous surface, or may be raised slightly above the surface of the carrier material (as in commercial cochlear implants) or may be located slightly below the surface of the carrier material (which may be called a recessed electrode contact).

A distal electrode 202 of the at least a first pair of electrodes may be located in proximity to a tip 230 of the implant 200, wherein the tip 230 may be inserted first into the electrically conductive volume. In such an example, the first inserted electrode, distal electrode 202, is used to determine an impedance measurement which in turn can indicate a distance between the electrode contact surface 210 (which comprises the surface of the distal electrode) and the volume in which the implant 200 is inserted.

The implant 200 may be a cochlear implant which is insertable into a scala tympani of a cochlea, in which case the electrically conductive volume is a cochlea, and the distance which can be determined is between the electrode contact surface and an inner wall of the scala tympani of the cochlea. In another example, the implant 200 may be a brain implant insertable into a portion of a brain. In this example, the electrically conductive volume is a brain, and the distance is between the electrode contact surface and an internal surface of the brain. In some examples, the distance may be determined to a high accuracy, for example, within +/- 0.1mm error or +/- 0.05mm error. In some examples, the distance determined may be used to indicate a region of the implantation environment, e.g., it may indicate (in the example of a cochlear implant) the modiolar, if the distance from the electrode to the wall is smaller or equal than 0.2mm; the middle, if the distance from the electrode to the wall is between 0.2 and 0.6mm; and the lateral if the distance is greater or equal than 0.6. Other examples may be envisaged.

Depending on which electrode pair(s) is/are used from the electrodes 202, 204, 206, 208, different impedance measurements may be taken. For example, a tetrapolar impedance may be measured using a first pair of electrodes of an electrode array acting as an inner pair and a second pair of electrodes acting as an outer pair. In the illustrated example of Figure 2, the inner pair is formed by a first pair of electrodes 204, 206 and the external pair is formed by a second pair of electrodes 202, 208. As another example, a bipolar impedance may be measured using a pair of electrodes (e.g. electrodes 202, 204, electrodes 202, 206, electrodes 202, 208, electrodes 204, 206, electrodes 206, 208).

The computer-readable instructions cause the apparatus 100 to receive 1008 a first impedance signal 108a from a first measurement pair of electrodes of the implant 200. The first impedance signal 108a is indicative of a first impedance due to interaction of the at least a first current pulse with the electrically conductive volume in which the implant 200 is inserted.

The computer-readable instructions cause the apparatus 100 to receive a second impedance signal 108b from a second measurement pair of electrodes of the implant 200. The second impedance signal 108b is indicative of a second impedance due to interaction of the at least a first current pulse with the electrically conductive volume. The first measurement pair of electrodes and the second measurement pair of electrodes are different.

It will be appreciated that the same first pair of electrodes of the implant 200 may be used to obtain different impedance measurements. For example, electrodes 204 and 206 may be used as electrodes used to generate the first current pulse. Electrodes 204 and 206 may be used to obtain a bipolar impedance measurement, and electrodes 202 and 208 may be used to obtain a tetrapolar impedance measurement. In some examples the same first current pulse may be used to trigger plural impedance measurements (such as a bipolar impedance measurement and a tetrapolar impedance measurement). In other examples, separate first current pulses may be used to trigger different impedance measurements (either from the same pair of measurement electrodes or from different pairs of measurement electrodes).

That is, the at least a first pair of electrodes may be a single pair of electrodes; and the at least a first electrode operation signal 106a may be a single electrode operation signal.

In some examples, the at least a first pair of electrodes may comprise a first pair of electrodes and a second pair of electrodes different from the first pair of electrodes; the at least a first electrode operation signal 106a, 106b may comprise a first electrode operation signal 106a and a second electrode operation signal 106b, and the at least a first current pulse generated on at least a first pair of electrodes may comprise a first current pulse generated on the first pair of electrodes and a second current pulse generated on the second pair of electrodes.

The apparatus 100 may be configured to receive the first impedance signal and the second impedance signal simultaneously in some examples.

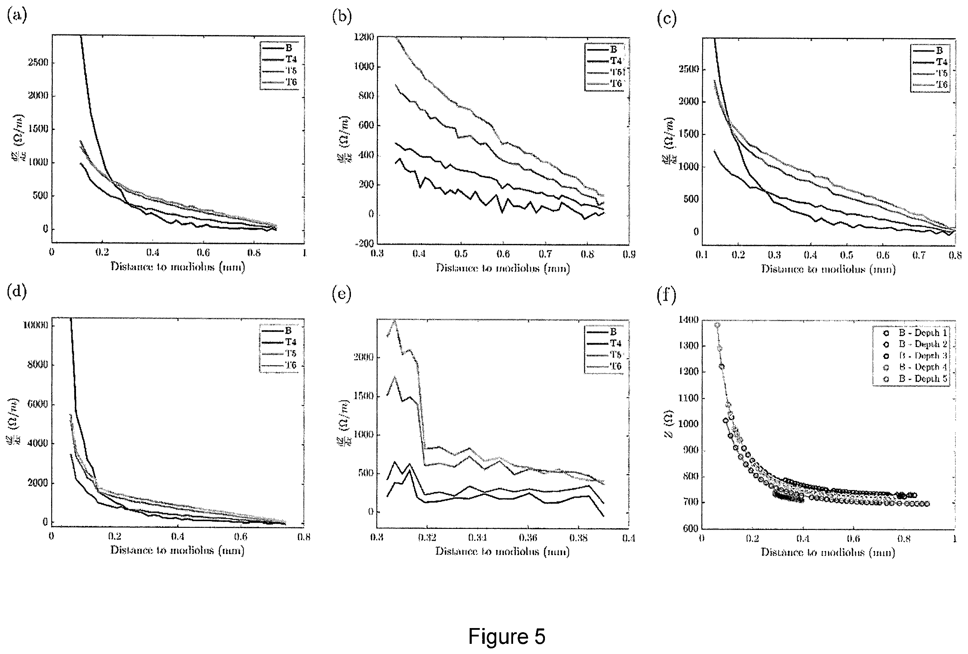

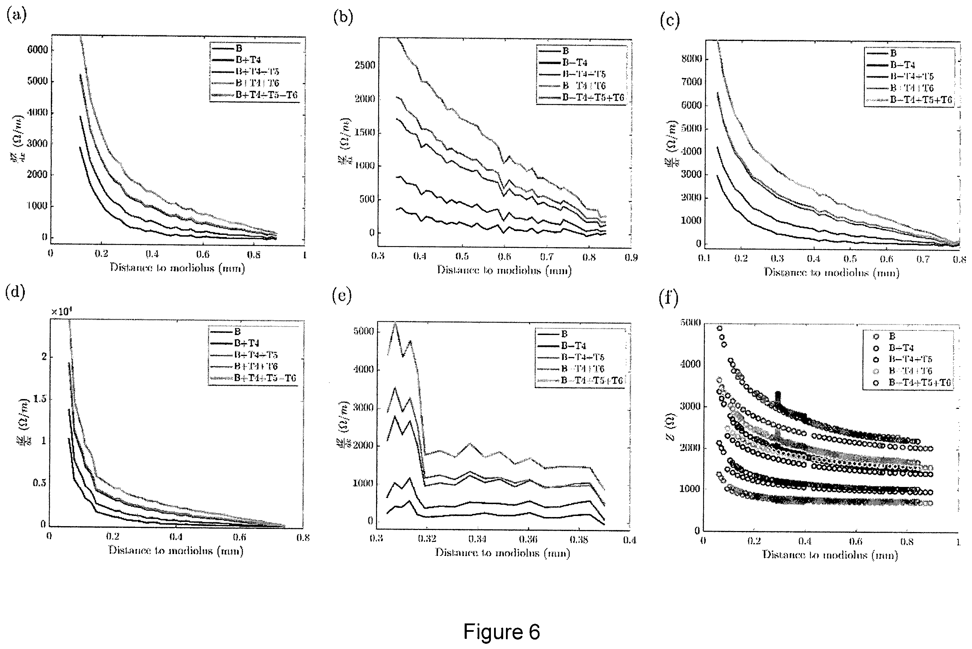

The computer-readable instructions cause the apparatus 100 to determine 1010, in dependence on the first impedance signal 108a and the second impedance signal 108b, and on a predetermined mapping relationship 150, a distance between the electrode contact surface 210 and a wall of the electrically conductive volume. The predetermined mapping relationship 150, as described later in relation to Figures 5 and 6, may be obtained from previous experiments and/or previous modelling of first and second impedance signals and a known distance between the electrode contact surface 210 and the wall of the electrically conductive volume in which the implant is inserted.

The computer-readable instructions cause the apparatus 100 to output 1012 the determined distance between the electrode contact surface and a wall of the electrically conductive volume. For example, as discussed below, the output may be indicated to a clinician to assist them in manual insertion of the implant, and/or to a robotic surgery apparatus as feedback to guide autonomous robotic implant insertion.

The first impedance signal 108a may be indicative of a tetrapolar impedance, received based on a voltage difference between the first measurement pair of electrodes. The first measurement pair of electrodes comprises a distal first measurement electrode positioned at a distal end of the first pair of measurement electrodes, and a proximal first measurement electrode positioned at a proximal end of the first pair of measurement electrodes; so the at least a first pair of electrodes are located between the distal and proximal first measurement electrodes. The second impedance signal 108b may be indicative of a bipolar impedance, received based on a voltage difference between the second pair of electrodes.

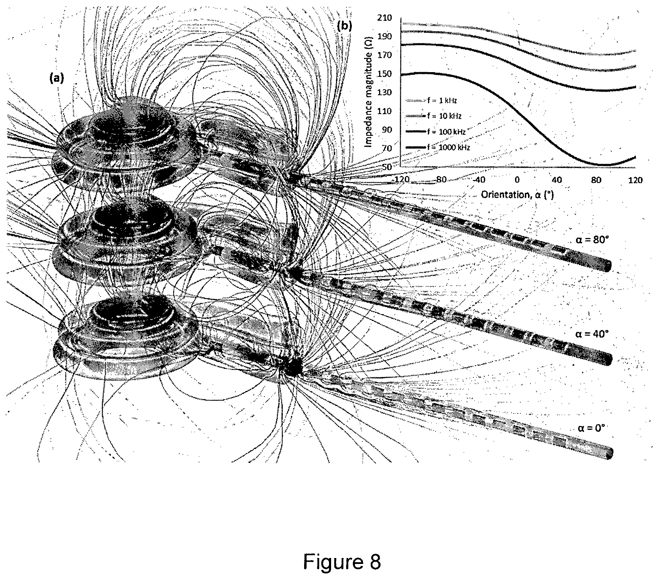

In some examples, the apparatus 100 may be further configured to determine 1014, in dependence on the first impedance signal 108a, the second impedance signal 108b, and the predetermined mapping relationship 150, a pose of a portion of the implant within the electrically conductive volume and output 1016 the determined pose. This is discussed in more detail in relation to Figure 8 relating to the effect of pose (rotation of the implant) within the volume.

The apparatus 100 may be further configured, in some examples, to determine, in dependence on the first impedance signal 108a, the second impedance signal 108b, and the predetermined mapping relationship 150, one or more of: intravolume pressure, implant insertion speed, insertion depth, risk of trauma to the wall of the of the electrically conductive volume, a level of trauma of the wall of the electrically conductive volume, a translocation of the implant through the wall of the electrically conductive volume, and occurrence of a tip roll-over event of the implant. These factors may be derived from the determined distance, and in some examples further from the determined pose, and/or information about the patient's anatomy. In some examples, as well as determining the distance between the electrode contact surface and a wall of the electrically conductive volume (and/or a pose of a portion of the implant within the electrically conductive volume and/or another factor as above) in dependence on the first impedance signal 108a, the second impedance signal 108b, and the predetermined mapping relationship 150, additional feedback may be considered. Thus the distance, pose, and/or other factor may be determined further in dependence on camera image feedback (e.g. photo or video images captured of the insertion environment) and/or on feedback from the surgical instrument used to perform the insertion (e.g. from one or more robotic elements or motors of the instrument).

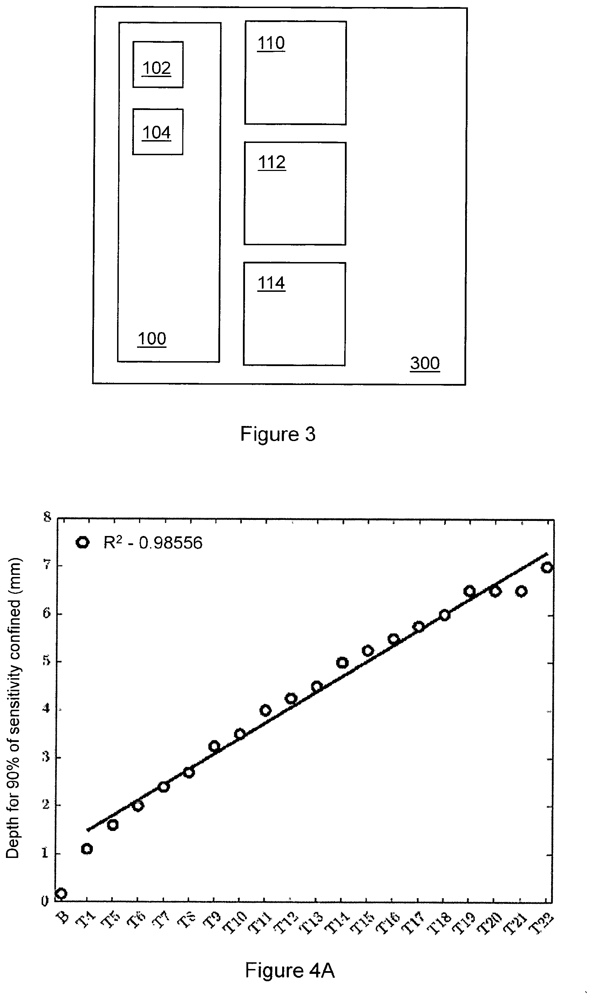

Figure 3 shows schematically a system 300 comprising an apparatus 100 according to examples discloses herein. The system 300 is for controlling insertion of an implant 200 into an electrically conductive volume in a patient. The system comprises an apparatus 100 as disclosed herein, and a pulse generator 110. The system 300 may further comprise a front-end amplifier 112 which is configured to receive the first impedance signal 108a and the second impedance signal 108b (e.g. simultaneously). The system 300 may further comprise a multiplexer 114 configured to select at least the first pair of electrodes and the second pair of electrodes from the plurality of electrodes 202, 204, 206, 208 of the implant 200.

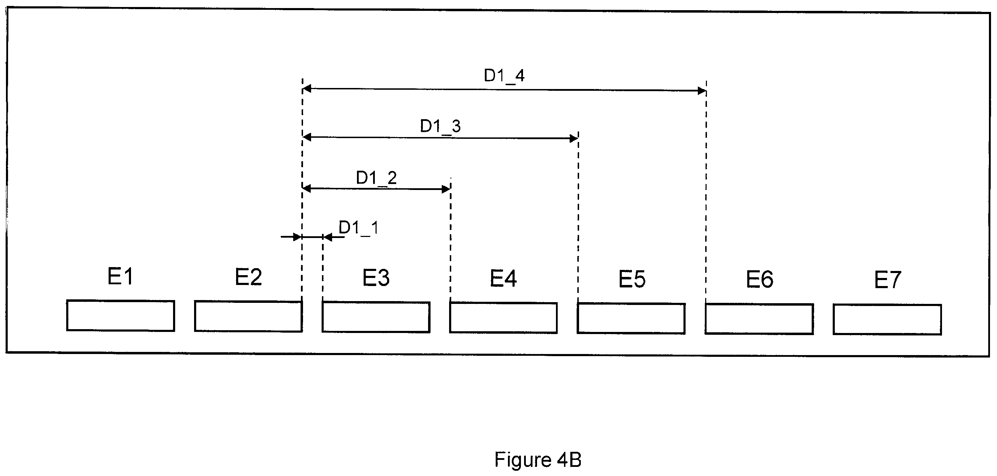

Figure 4A shows data indicating a depth of implant insertion at which 90% sensitivity is confined for tetrapolar and a bipolar electrode combinations. Figure 4B illustrates schematically a series of electrodes E1 to E7 and some distances between respective electrode pairs D1_1 to 4. Figure 4A shows that the sensor sensitivity increases linearly with the separation between electrode pairs. Regarding sensing depth, for example, if the sensing depth value is 2mm, it means that the sensor can sense when is touching the wall (0mm), or when is 2 mm from the wall. Beyond this point the sensing output may not be accurate or realistic.

In all the measurements shown in Figure 4A, each impedance measurement is compared with the distance from a point in the electrode array to the region of interest. A bipolar measurement is illustrated as point "B". Tetrapolar measurements T4 to T22 are indicated. As the T number on the horizontal axis increases so does the spacing between the inner electrodes. For example, "T13" is a measurement from two external electrodes., two inner electrodes, and nine electrodes (not used for the T13 measurement) between the two inner electrodes. A combination of detecting both tetrapolar and bipolar impedance may be advantageous to obtain a high sensitivity to distance from electrode surface to implant volume wall for both larger distances, and smaller distances.

Tetrapolar impedance may be measured using, for example, the first four electrodes of an electrode array (e.g. a cochlear implant electrode array); these first four electrodes are labelled as 202, 204, 206, 208 in Figure 2 and as T4 ("tetrapolar" of the first "4" electrodes of the implant) in Figure 4A. To determine the tetrapolar impedance, a current source may be connected to the two outer electrodes (which may be called an external pair of electrodes), and the voltage difference may be measured between a pair of electrodes inside the outer electrodes (these may be called an internal pair of electrodes). From Figure 2, the outer electrodes may be electrodes 202 and 208 and the inner electrodes may be electrodes 204 and 206. An alternative combination of electrodes where the current source is connected to the internal two electrodes 204, 206 and impedance is measured between the external pair of electrodes 202, 208 should yield the same result, since reciprocity applies.

The voltage may then pass through a front-end amplifier 112, and used to acquire tetrapolar measurements. The front-end amplification may improve accuracy of impedance measurement.

In an autonomous robotic surgery system, the received impedance signals may be subsequently used as an input in a microcontroller which processes these measurements, determine an implant - volume wall distance and update the insertion parameters of the robotic surgery system.

Looking again at Figure 4A, a further tetrapolar impedance may be measured using electrodes 202, 204, 208, 209 (and not electrode 206) of the electrode array / cochlear implant electrode array, which is shown as point T5 in Figure 4A. The inner pair of electrodes is formed by electrodes 204 and 208, and the external pair of electrodes by electrodes 202 and 209. In this case, the sensitivity depth as shown on the vertical axis is higher compared to the previous case using electrodes 202, 204, 206, 208. For this to be valid, the distance between each pair of electrodes has to be the same as discussed below in relation to Figure 4B. The gap / separation between the inner pair of electrodes can be increased for additional sensitivity depth, as shown in Figures 4A and 4B.

Example tetrapolar arrangements as disclosed herein may be understood by considering the Geselowitz sensitivity theorem. The theorem states that injecting and measuring electrodes can be interchanged without a change in measured impedance (reciprocity) and provides a measure of sensitivity, S, to impedance changes that can be expressed as S= (J_1 · J_2)/ (I_1 I_2), where J_1 is the current density when current I_1 is injected between the outer pair of electrodes and J_2 is the current density when I_2 is injected between the inner pair of electrodes. The magnitude of S indicates the contribution of a region within a volume conductor to the measured impedance. Hence, to understand the depth to which examples disclosed herein can sense, a confinement to 90% of the total sensitivity is used as a sensible measure of sensitivity. As shown in Figure 4A and Figure 4B, this depth increases with the distance (D1) between the inner pair of electrodes. As D1 increases, the potential difference also increases (as there is a larger resistance due to the greater distance) and as a result the signal-to-noise ratio also increases.

The Geselowitz sensitivity theorem may also be used to understand the operation of bipolar arrangements disclosed herein, in which case the two electrode sets are the same and thus S=J_1 · J_1 / I_1^2. The sensitivity, S, as defined here, is the dot product of two vectorial quantities and is thus a scalar (i.e., it can have positive and negative values). If all the electrodes are separated by the same distance, as show in Figure 4B, the pair of electrodes on the left and on the right are separated by the same distance. In T4 the electrodes on the left may be E1 and E2 and the electrodes on the right may be E3 and E4. In T5 the electrodes on the left may be E1 and E2 and the electrodes on the right may be E4 and E5. In this example, a linear relationship may be shown between D1 (i.e. the separation between the electrode pair on the left and the electrode pair on the right) and sensitivity depth. If the separation between electrodes is not the same, this relationship may not hold and in such an example, each combination of tetrapolar electrodes may be modelled to calculate the sensitivity depth.

Thus, as mentioned above in relation to the electrodes operated by the apparatus 100, the first impedance signal 108a may be indicative of a tetrapolar impedance obtained from a first measurement pair of electrodes 202, 208. The second impedance signal 108b may be indicative of a bipolar impedance, received based on a voltage difference between a second pair of electrodes 204, 206 between the first pair of electrodes 202, 208. The second impedance signal 108b may be indicative of a further tetrapolar impedance obtained from a second measurement pair of electrodes 202, 209. It may be envisaged that different combinations of electrodes may be used to obtain the first and second (and possibly further) impedance signals 108a, 108b.

In some examples, more than one measurement (to obtain first and second impedance measurements) may be taken; for example, as the insertion of the implant progresses there may be periodic checks to monitor the insertion process. Therefore, in some examples, with reference again to Figure 1, the apparatus 100 may be configured to transmit, to the pulse generator 110, a first further electrode operation signal 106c configured to generate a first further current pulse on a further pair of electrodes of the implant 200, wherein the further pair of electrodes comprises at least one different electrode to the at least a first pair of electrodes so the further pair of electrodes are separated by a different amount than the at least a first pair of electrodes. The apparatus 100 may be configured to receive a first further impedance signal 108c indicative of a first further impedance due to interaction of the first further current pulse with the electrically conductive volume, and determine the distance between the electrode contact surface 210 and a wall of the electrically conductive volume further in dependence on the first further impedance signal 108c.

The apparatus 100 may be further configured to: transmit, to the pulse generator 110, a second further electrode operation signal 106d configured to generate a second further current pulse on a second further pair of electrodes of the implant, wherein the second further pair of electrodes comprises at least one different electrode to the at least one first pair of electrodes so the second further pair of electrodes are separated by a different amount than the at least one first pair of electrodes; receive a second further impedance signal 108d indicative of a second further impedance due to interaction of the second further current pulse with the electrically conductive volume; and determine the distance between the electrode contact surface 210 and a wall of the electrically conductive volume further in dependence on the second further impedance signal 108d.

The apparatus 100 may be configured to transmit the first further electrode operation signal 106c and the second further operation electrode signal 106d to the same pair of electrodes, so the first further pair of electrodes is the same as the second further pair of electrodes. For example, a pair of electrodes 204, 208 may be used to transmit a further current pulse. The first further pair of electrodes 202, 209 may indicate a tetrapolar impedance and the second further pair of electrodes 204, 208 may indicate a bipolar impedance, which for example are different to tetrapolar and bipolar impedances obtained from first and second electrodes.

In some examples, the apparatus 100 may be operated to obtain a tripolar impedance whereby a first electrode (for example, electrode 202) is used for both transmitting a current pulse and measuring an impedance, a second electrode (for example, electrode 204) is used with the first electrode to transmit the current pulse, and a third electrode (for example, electrode 206) is used with the first electrode 202 to measure the current pulse, where the first 202, second 204, and third 206 electrodes are all different. In other words, the apparatus 100 may be further configured to: transmit, to the pulse generator 110, a third electrode operation signal configured to generate a third current pulse on a third pair of electrodes 202, 204 of the implant 200; and receive a third impedance signal indicative of a third impedance due to interaction of the third current pulse with the electrically conductive volume, received based on a voltage difference between a third measurement pair of electrodes 202, 206, the third measurement pair of electrodes 202, 206 comprising: a common third electrode 202 of both the third pair of electrodes 202, 204 and the third measurement pair of electrodes 202, 206; and a further third electrode 206 of the electrode array, the further third electrode 206 different from the third pair of electrodes 202, 204.

The further third electrode 206 may be, for example, one of the at least a first measurement pair of electrodes used to measure a tetrapolar impedance or bipolar impedance; or an electrode located between the at least a first measurement pair of electrodes.

Thus in some examples, combined tetrapolar, tripolar and bipolar impedance measurements may be performed. The measurements may be taken using a single current source in some examples. To calculate tripolar impedance measurements, the voltage difference between the electrode where the current is injected and any electrode between the electrodes used to measure tetrapolar impedance measurements, both inclusive, may be used. The combined impedance may be compared with the distance from a point in the electrode array to the region of interest. Other pair of combinations between the three types of impedance measurements may also be made.

In some examples, the apparatus 100 may be operated to obtain a monopolar impedance, whereby one electrode of the implant 200 and a separate/reference electrode 240 separate from an implant electrode are used. The apparatus 100 may thus, in some examples, be further configured to: transmit, to the pulse generator 110, a fourth electrode operation signal configured to generate a fourth current pulse on a fourth pair of electrodes (for example, 202) and 240, the fourth pair of electrodes comprising a separate/reference electrode 240 external to the electrodes of the implant, and a fourth electrode (e.g. electrode 202) of the electrodes of the implant 200; and receive a fourth impedance signal indicative of a fourth impedance due to interaction of the fourth current pulse with the electrically conductive volume, received based on a voltage difference between the fourth pair of electrodes (in this example, electrode 202 of the implant and reference electrode 240).

In some examples, combined monopolar, and tetrapolar and/or tripolar and/or bipolar impedance measurements may be made. A current source may be connected between the electrode of interest and a separate/reference electrode 240 to calculate monopolar impedance measurements (the separate/reference electrode 240 may also, in some examples, be used to calculate tripolar, and/or tetrapolar impedance). The voltage difference between the electrode where the current is injected and the named reference may be measured. The combined impedance may be compared with the distance from a point in the electrode array to the region of interest. Other pair and trio combinations between the four types of impedance measurements are also envisaged.

To obtain different measurements from the implant 200 and ascertain at least a distance between the electrode contact surface 210 and the wall of the volume into which the implant is inserted, as above, various different combination of electrodes may be used to transmit current pulses and obtain impedance measurements. Advantageously, the same electrodes may be used to transmit a current pulse and different electrodes may be used to simultaneously detect impedance; for example, both bipolar and tetrapolar impedance may be detected simultaneously from the same input current pulse on an electrode pair.

Figures 5a) to e) shows change in bipolar and tetrapolar impedance measurements with electrode-wall distance at five depths of insertion of a cochlear implant (e.g., derivative of the impedance with respect to electrode-wall distance). The gradient is inverted. Figure 5f) shows the bipolar impedance measurement at those depths, for a plurality of combinations of electrodes. The wall in this example is the modiolar and the distance is the electrode-modiolar distance. Data such as this may be used as the predetermined mapping relationship 150 from which to obtain distance information when performing a subsequent insertion procedure. In the legends on the figures and with reference to Figure 2, B: Bipolar between electrodes one 202 and two 204. T4: Tetrapolar using electrodes one 202 and four 208 as injecting and two 204 and three 206 as measuring. T5 Tetrapolar using electrodes one 202 and five 209 as injecting and two 204 and four 208 as measuring. T6: Tetrapolar using electrodes one 202 and six 211 as injecting and electrodes two 204 and five 209 as measuring. In this example, a current source is connected to the inner pair. The voltage difference between the external pair is used to calculate the tetrapolar impedance, while the voltage difference between the internal pair is used to calculate the bipolar impedance. Impedance measurements are acquired as in the previous point. The gap between the inner pair can be increased for additional sensitivity depth, as described in 1). Each combined impedance measurement is compared with the distance from a point in the electrode array to the region of interest.

It can be seen, in particular in Figures 5a, 5c and 5d, that at smaller distances, less than approx. 0.25 mm from the modiolus, the bipolar impedance provides a more sensitive detection of distance because there is a greater variation in impedance for each unit change of distance using bipolar impedance rather than tetrapolar impedance at these smaller distances. However, at larger distances, greater than approx. 0.25 mm from the modiolus, the tetrapolar impedance provides a more sensitive detection of distance because there is a greater variation in impedance for each unit change of distance using tetrapolar impedance rather than bipolar impedance at these larger distances (there is also a higher impedance signal magnitude for tetrapolar impedance rather than bipolar impedance at distances greater than about 0.25 mm, which may make tetrapolar impedance easier to detect and measure than bipolar impedance at these larger distances). Thus, by using a combination of impedance types, an improvement in sensitivity to the distance may be achieved over a range of distances.

Figures 6a) to e) shows change in combined bipolar and tetrapolar impedance measurements with electrode-wall distance at five depths of insertion (e.g., derivative of the impedance with respect to electrode-wall distance), and Figure 6f) shows the combined bipolar and tetrapolar impedance measurement at those depths, for a plurality of combinations of electrodes. The gradient is inverted. That is, the bipolar and tetrapolar impedances are added together from a plurality of combinations of measurement electrodes as the implant is inserted. The wall in this example is the modiolar and the distance is the electrode-modiolar distance. In the legends on the Figures and with reference to Figure 2, B: Bipolar between electrodes one 202 and two 204. T4: Tetrapolar using electrodes one 202 and four 208 as injecting and two 204 and three 206 as measuring. T5 Tetrapolar using electrodes one 202 and five 209 as injecting and two 204 and four 208 as measuring. T6: Tetrapolar using electrodes one 202 and six 211 as injecting and electrodes two 204 and five 209 as measuring.

As in Figures 5a-5e, it can be seen (particularly in Figure 6f) that the greater sensitivity at larger distances of the tetrapolar impedance, in combination with the greater sensitivity at smaller distances of the bipolar impedances, allow for an improved sensitivity measurement of the distance of the modiolus from the electrode contact surface 210 of the implant 200.

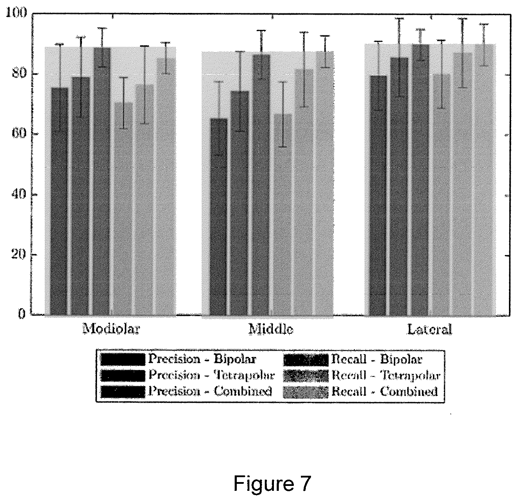

By using combined tetrapolar and bipolar impedance measurements, the implant localisation accuracy, sensitivity and precision increase. Figure 7 shows a bar graph of precision and sensitivity for three sensing modalities (bipolar, tetrapolar, and bipolar+tetrapolar) obtained from the example of Figure 5a-f and 6a-f. It can be seen that the tetrapolar impedance provides a more accurate distance determination that the bipolar impedance, and that a combination of bipolar and tetrapolar impedance measurement provides a still more accurate determination. In the legend to Figure 7, "Precision" is the proportion of all the model's positive classifications that are actually positive and "Recall" is the proportion of all actual positives that were classified correctly as positives. For this classification, the electrode-modiolar distance was divided into three zones: modiolar (i.e., closest to the wall), middle and lateral. Extracted from Figure 7 are the following data illustrating electrode-modiolar distance detection accuracy for the three sensing modalities: bipolar, tetrapolar and combined.

| | **Bipolar** | **Tetrapolar** | **Combined** |
|---|---|---|---|
| Mean Accuracy | 74.4% | 83.8% | 90.6% |
| Standard Deviation of Accuracy | 4.2% | 2.5% | 1.8% |
| p-value Comparison of Bipolar and Tetrapolar | | 4.2 · 10⁻⁵ | |
| p-value Comparison of Bipolar and Combined | | 8.5 · 10⁻⁷ | |
| p-value Comparison of Tetrapolar and Combined | | 1.7 · 10⁻⁴ | |

Mean and standard deviation over 10 iterations.

Table 1 - Electrode-modiolar distance detection accuracy for the three sensing modalities: bipolar, tetrapolar and combined (i.e., bipolar+tetrapolar).

The predetermined mapping relationship 150 in some examples may be obtained from data such as that presented in Figures 5a-f and 6a-f. In some examples, the predetermined mapping relationship may be determined using a machine learning model trained using data such as that presented in Figures 5a-f and 6a-f; in other words, obtained from first impedance signal training data (e.g. bipolar), second impedance signal training data (e.g. tetrapolar), and training data distances between the electrode contact surface and a wall of the electrically conductive volume corresponding to the first and second impedance signal training data. For example, a machine learning module may be coupled to a robotic insertion device controller and collect impedance measurements such as those illustrates in Figures 5a-f and 6a-f (all the combinations) at different frequencies over time, and may learn to classify the distance from a point in the electrode array to the region of interest. The ML model may then output proximity values to the structure of interest based on the impedance. The specific anatomy and features of the patient, with its particular scala tympani shape and the tissue layers surrounding it, may be used as inputs in this module to improve the position and event detection accuracy. In some examples, training data may comprise depth of insertion, (i.e., how deep the implant is inserted in the conductive volume); distances of other electrodes to the wall, area between these and other electrodes and the wall, implant dimensions, electrodes topology, and/or volumetric information of the conductive medium.

In some examples, the apparatus 100 may be configured to determine the predetermined mapping relationship 150. The apparatus may be configured to calibrate the at least a first current pulse in dependence on anatomical and/or physiological data of the patient to obtain the predetermined mapping relationship. The anatomical and/or physiological data may be obtained from the patient preoperatively. Intraoperatively, initial impedance measurements may be taken to calibrate the predictive model for that specific patient. These initial measurements can be taken before insertion and after the surgeon has positioned the implant with respect to the entry point in the cochlea. These initial measurements can be taken during insertion, once that the surgeon has started the insertion and the implant has entered the conductive volume (e.g. the cochlear).

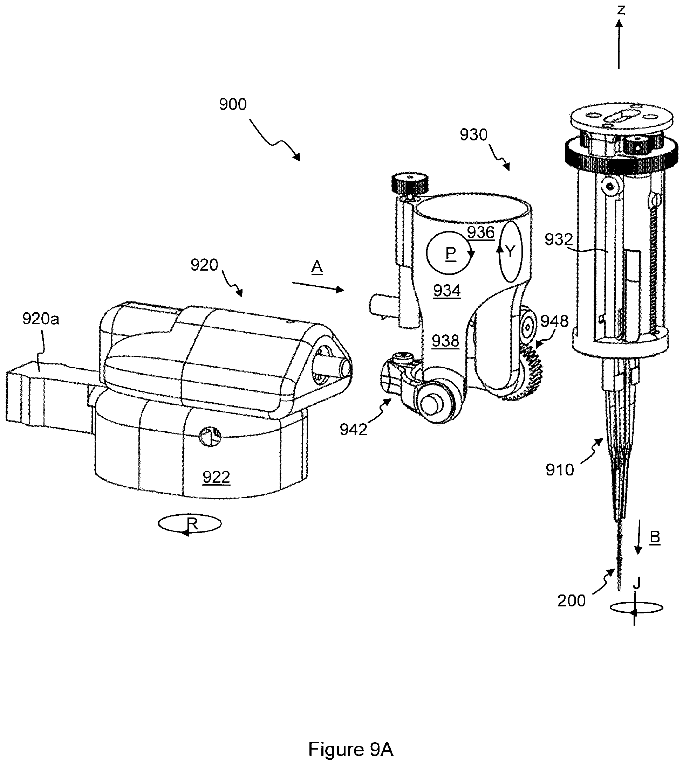

The apparatus 100 may be configured to output the determined distance between the electrode contact surface and a wall of the electrically conductive volume to an output device configured to provide an indication of the output to a user (e.g. to guide a surgeon in performing the implant insertion). The apparatus 100 may be configured to output the determined distance between the electrode contact surface and a wall of the electrically conductive volume to a surgical instrument controllable using the output to perform insertion of the implant into an electrically conductive volume in a patient. This is described in more detail with reference to Figures 9A-9J.

The output determined distance (i.e., the distance from the electrode to the wall) may be used to create a visualisation of the position of the implant in the conductive volume in real-time in an output device. The output determined distance may be used to visualise implant positioning and factors such as: translocation, tip roll over, insertion speed, insertion depth, intravolume pressure, and risk of trauma to the wall of the of the electrically conductive volume. The output determined distance as an input to tune the insertion parameters of a robotic system (such as that of Figures 9A-9J) controlling the implant insertion autonomously. The output determined distance may be used as an input to generate a feedback signal for the surgeon in a controller device used to control robotic system.

Figure 8a-b shows a model of electrical potential field in a cochlea and electrode array with changes in impedance with electrode rotation angles and electrical frequencies. Figure 8(a) shows a surface illustration of the electric potential field in the cochlea and electrode array (EA) model at implant rotation angles α (which determine the surface which the electrode contact surface 210 is facing). Implant rotation angles α = 0°, 40° and 80° are illustrated. Tubes represent the current density.

Figure 8(b) is a plot showing the change in impedance magnitude for different EA angles (α between -120 °and 120°) and stimulation frequencies (1kHz, 10kHz, 100kHz and 1000kHz). In some examples, multiple current pulse frequencies may be used to increase the accuracy of location and event detection. Changes in impedance determined to be higher at higher frequencies. It can be seen than the impedance magnitude changes with rotation angle and this variation is magnitude is higher (while the absolute magnitude decreases) at higher frequency.

Thus, a frequency of the current pulse may be changed to obtain additional positional information of the implant in the volume. For example, the apparatus 100 may be configured to: transmit one or more of a plurality of first electrode operation signals each at a different frequency, and a plurality of second electrode operation signals each at a different frequency; and determine the distance between the electrode contact surface and a wall of the electrically conductive volume in dependence on one or more of: a plurality of first impedance signals respectively received due to the plurality of first electrode operation signals, and a plurality of second impedance signals respectively received due to the plurality of second electrode operation signals.

Therefore, whereas monopolar or bipolar impedance measurements are known to localise an electrode array and to detect events during insertion, these methods do not provide the necessary accuracy. The examples disclosed herein use tetrapolar impedance measurement, for example in combination with other impedance measurements, to improve the accuracy of localisation and adverse event detection (e.g. tip roll over). Unlike existing technologies, examples disclosed herein may be used to collect measurements at different frequencies, to further improve system accuracy. The following discussion focuses on an acquisition system disclosed herein which may be used to accurately read such impedance measurements.

Figures 9A to 9J show an example surgical instrument 900 according to examples disclosed herein. The surgical instrument 900 may be used to assist (e.g. cochlear) implant electrode array insertion. The instrument 900 can be used to gently grab and insert the implant electrode array. The instrument 900 may be motor-driven, and may be used for robotically assisted manipulation of an implant.

The insertion of the electrode array may be performed autonomously in some examples (for example controlled using apparatus described above), and in other examples, a surgeon may use the instrument 900, for example to pause autonomous operation at any point to continue implant insertion manually.

Examples disclosed here provide a compact and reusable robotic insertion instrument 900 for implant surgery (such as cochlear implant surgery and deep brain stimulation surgery), which features a single design / arrangement capable of inserting a range of commercially available implants. Examples also include features which provide advantages over known systems such as the implant clamping mechanisms, modular structure with quick release, implant guide, and insertion module arranged to improve the field of view of the region of surgery accessible through a microscope.

The instrument 900 of Figures 9A to 9J is a surgical instrument. The surgical instrument may be suitable for implant insertion. The following discussion focuses on the use of the instrument 900 used for implant positioning and/or implantation/insertion. However, it will be appreciated in other examples within the scope of this disclosure, the instrument 900 may be used for the purposes of surgical tool manipulation e.g. positioning and movement. The discussion herein in relation to insertion, positioning and/or movement of an implant may be understood to apply to the positioning and movement of a surgical tool (e.g. a needle or probe) held in the instrument, rather than an implant. Therefore, the term "insertion module" 930 used herein may be replaced with the term "handling module" 930, "positioning module" 930 or "manipulation module" 930. The discussion of an "insertion module" or similar may also be understood to apply to held tools (and implants in some examples) where they may not necessarily be "inserted" but may be positioned, and as such any "insertion" element may be labelled a "positioning" element. An example is locating a camera or other visual probe, or a supporting element for another tool, located outside the subject.

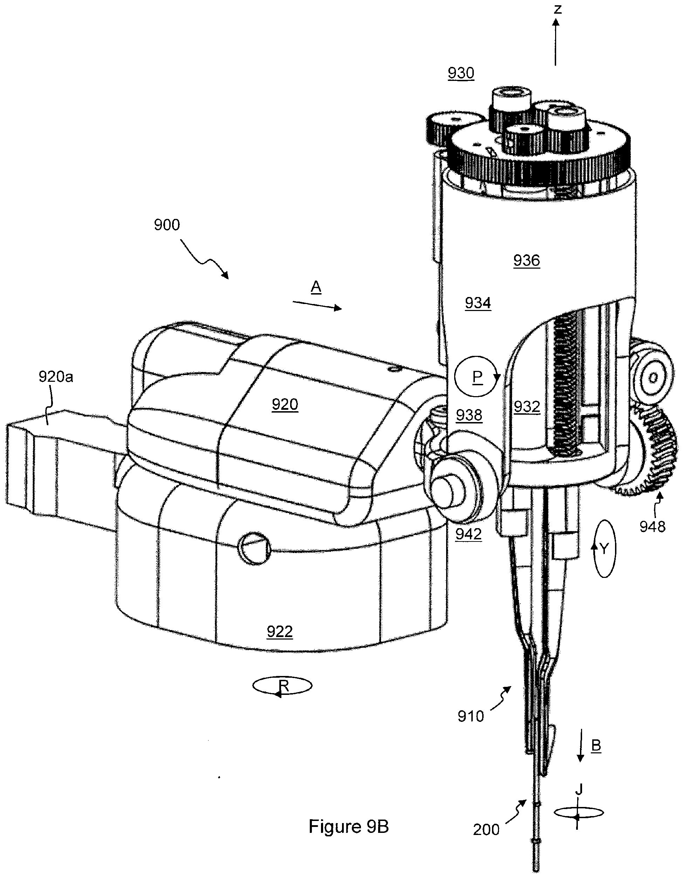

The instrument 900 comprises a positioning module 920 and an insertion module 930. The instrument may comprise an implant holder 910 or may comprise an implant holder connector as part of the insertion module 930 via which an implant holder 910 may be held/secured. The instrument 900 is modular (the implant holder 910, positioning module 920 and insertion module 930 may be separated from each other) and is compact. The design of the insertion module 930 proximal to the patient when in use allows for an improved field of vision of the surgery location, for example for a microscope to be able to image the surgery location and follow the insertion of an implant. Figure 9A shows an exploded view in which the positioning module 920 is separate from the insertion module 930, and the insertion module 930 itself is shown in the inner portion in which the implant holder 910 is located, separate from the outer portion which connects to the positioning module 920. Figure 9B shows the same instrument as in Figure 9A in the compact / assembled (non-exploded) form.

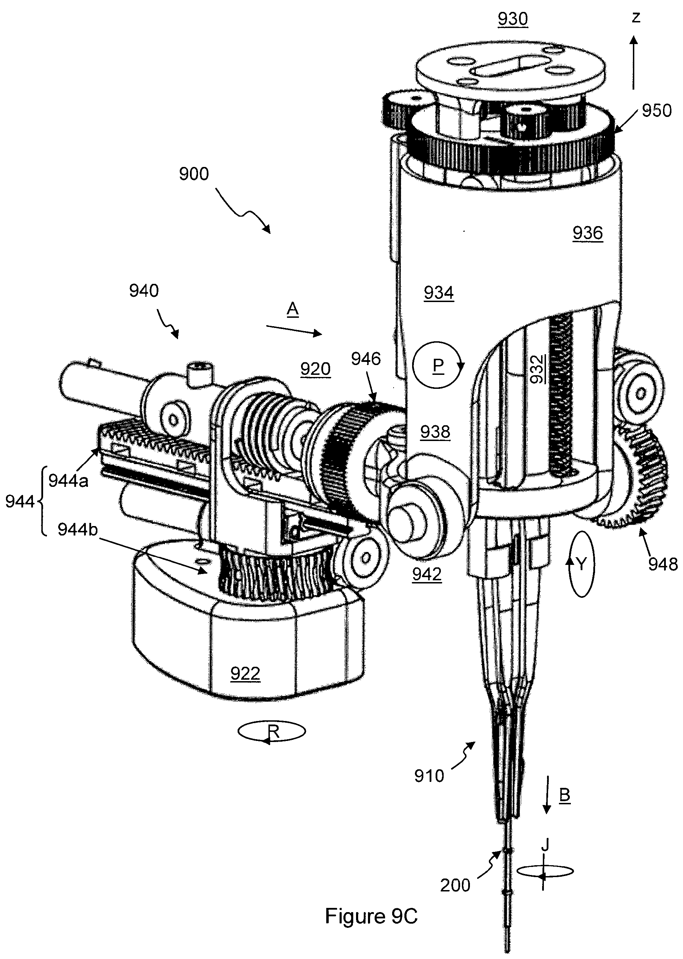

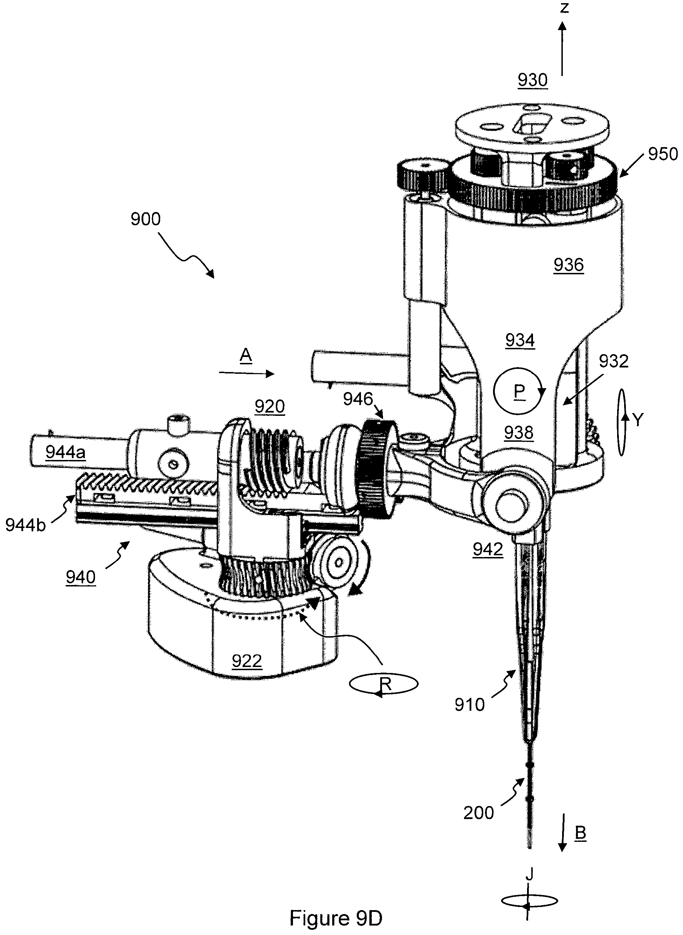

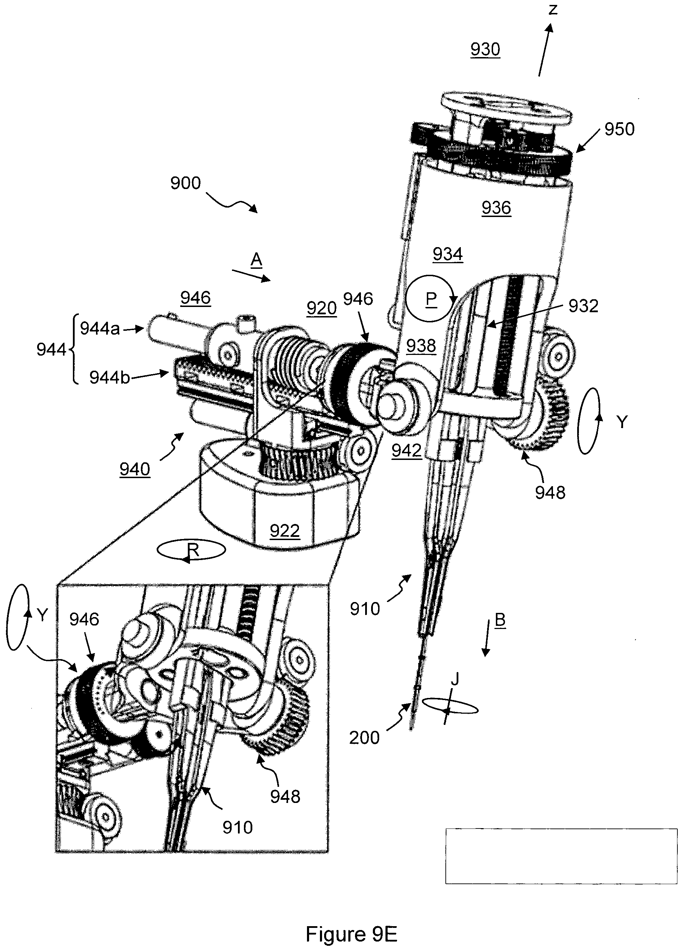

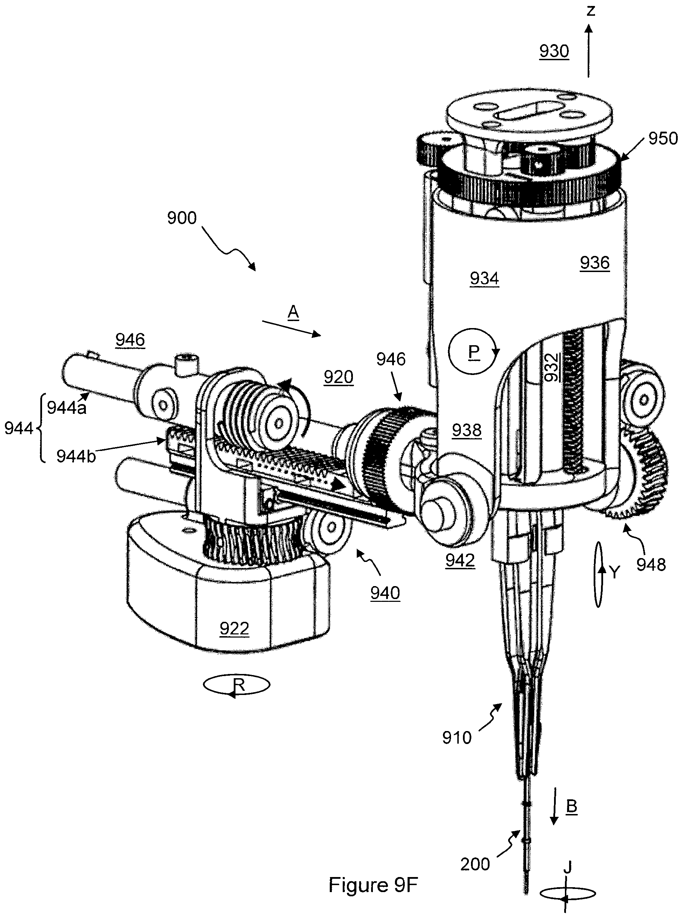

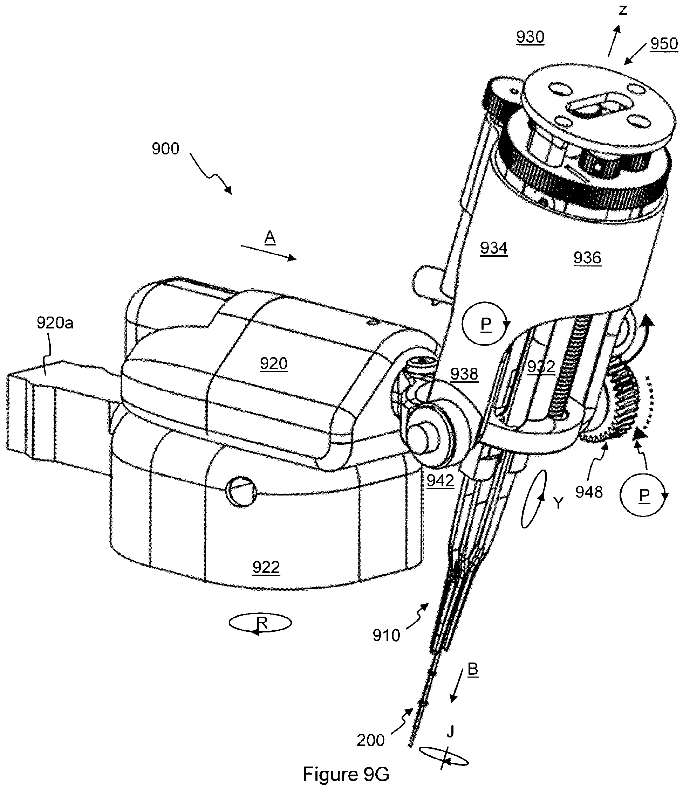

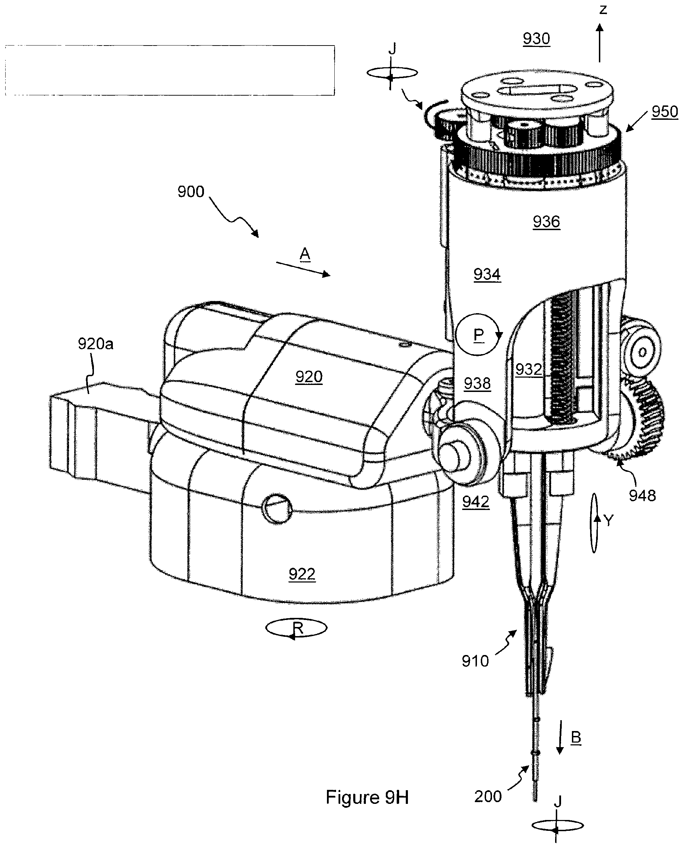

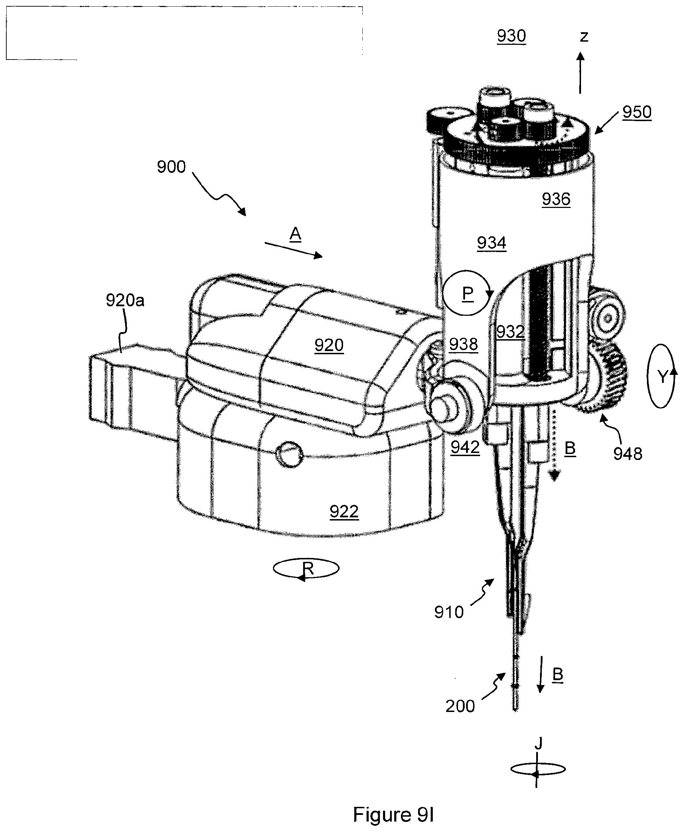

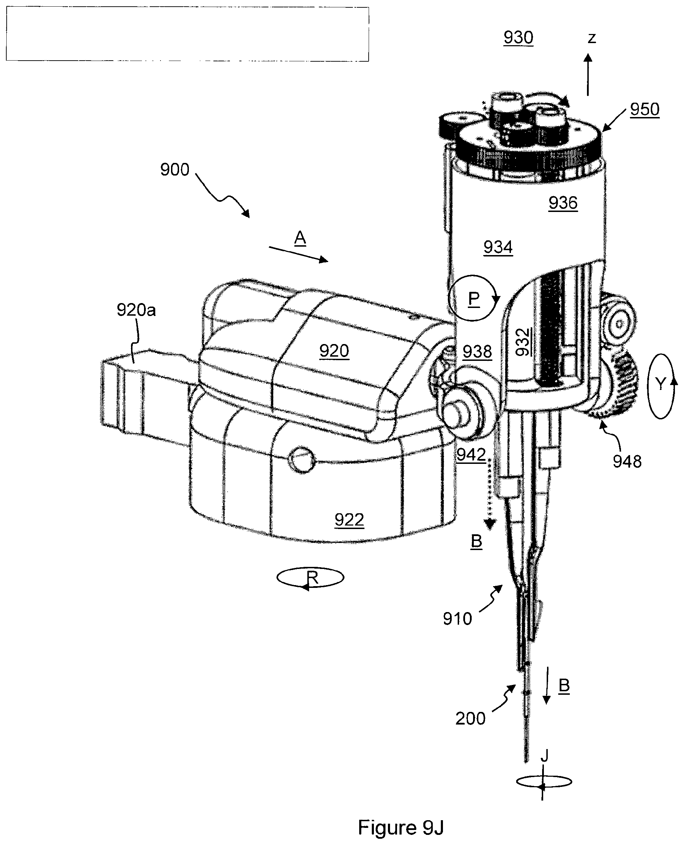

The surgical instrument 900 comprises: an insertion module 930; a linear translation portion 944a configured to translate the insertion module along a first linear direction "A" to change an entry position of implantation (as shown in Figure 9F compared with Figure 9C); a rotational translation portion/rotational gear mechanism 944b configured to rotationally translate the insertion module in a first plane of rotation "R" to change the entry position of implantation (as shown in Figure 9D compared with Figure 9C); a first rotatable portion 946 configured to rotate the supported insertion module in a yaw plane "Y" to change the angular direction of implantation (as shown in Figure 9E compared with Figure 9C); a second rotatable portion 948 configured to rotate the insertion module in a pitch plane "P" to change the angular direction of implantation (as shown in Figure 9G compared with Figure 9C), the pitch plane perpendicular to the yaw plane; and an implant holder movement portion 932 configured to: translate an implant holder 910 held in the insertion module 930 along a direction of implantation "B" (as shown in Figure 9I and 9J compared with Figure 9C) and rotate the implant holder 910 about an implant axis of rotation "J" along the direction of implantation (as shown in Figure 9H compared with Figure 9C).

In the examples shown, the implant holder 910 is configured to releasably hold an implant 200 for insertion into a volume. The insertion module 930 may comprise an easy access portion (for example formed by the arms/corresponding support element 938 and space therebetween) to connect / plug in a single-use implant holder 910 responsible for clamping the implant. In some examples inner portion may be removed from and replaced in the outer portion, for example, to change implant holder 910 between uses.

In these examples, the positioning module 920 comprises a patient-attachable portion/rotary base 922 configured to attach to a patient (e.g. the lower part of the positioning module 920 as shown may be positioned and attached to a patient). The positioning module 920 may be bone mounted (for implantation in a patient's head) to the patient's skull, for example using one or more screws (e.g., self-tapping) in such examples. The positioning module 920 may be attached to a robotic, active, or passive arm, for example, attached to the patient's bed, in some examples. This may provide additional stability when used in combination with attaching the positioning module 920 to the patient. The positioning module 920 may be thought of as a base, and assists in positioning the implant in the horizontal plane (as illustrated in Figures 9A-9J; more broadly it assists positioning of the implant 200 transverse to the direction of implantation).

The illustrations in Figures 9A-9B and 9G-9J show the positioning module with a housing, for example to help protect the mechanisms within the housing and to assist in maintaining a clean environment by the housing being cleanable. Figures 9C-9F show an example movement mechanism which may be used within the positioning module 920 to move the instrument 900 as described below.

As shown in Figures 9C-9F, the positioning module 920 comprises an insertion module mechanism 940 which may be used to position the insertion module 930. The instrument 900 comprises an insertion module support 942. This may be a insertion module support 942 (as shown, a semi-annular, half-loop, or C-shaped bracket, to which the housing 934 is mounted) which is part of the insertion module 930 as shown in Figure 9A which connects to a corresponding support element 938 of the positioning module 920. In other examples the insertion module support 942 may be part of the positioning module 920 to which the housing 934 of the insertion module 930 can be connected and disconnected. The insertion module support 942 is configured to support the insertion module 930. It may be thought of as a wrist joint providing a connection point between the positioning module 920 and the insertion module 930.

As shown in Figures 9C-9F, the positioning module 920 (i.e. the insertion module mechanism 940 thereof) comprises a translation portion 944. The translation portion 944 is configured to translate the supported insertion module 930 along a first linear direction "A" to change an entry position of implantation of the implant 200 into the patient as shown in Figure 9F. The linear movement may be facilitated by a prismatic joint driven by a rack and pinion mechanism or a linear translation portion 944a mounted over the patient-attachable portion/rotary base 922 as illustrated, though in other examples a different linear movement mechanism may be used.

As shown in Figures 9C-9F, the translation portion 944 is also configured to rotationally translate the supported insertion module in a first plane of rotation, shown as plane "R", to change the entry position of implantation, as shown in Figure 9D. The rotational translation movement may be facilitated by a rotational translation portion/rotational gear mechanism 944b mounted over the rotary base/patient-attachable portion 922 as illustrated, though in other examples a different rotational translation movement mechanism may be used.

Thus by way of the translation portion 944, the position of the implant 200 e.g. to be at the entry point for insertion, may be controlled.

As shown in Figures 9C-9F, the positioning module 920 (i.e. the insertion module mechanism 940 thereof) comprises a first rotatable portion 946. The positioning module 920 (i.e. the insertion module mechanism 940 thereof) is also configured, by way of the first rotatable portion 946, to rotate the supported insertion module 930 in a plane of rotation shown as plane "Y", to change the entry position / angle of implantation as shown in Figure 9E. As shown, for example, a rotational gear 946 may be used to vary the rotational angle of the implant 200 and thus the angle of insertion of the implant with respect to a volume. That is, there is a first rotatable portion 946 configured to rotate the supported insertion module 930 in a yaw plane "Y" to change the angular direction of implantation.

The positioning module 920 (i.e. the insertion module mechanism 940 thereof) may thus comprise motors to actuate movement in the linear direction "A" for translation, rotationally in the "R" plane for positional adjustment, and rotationally in the rotation plane "Y" for angular rotation. For example, there may be a linear motor configured to control movement of the rotary base 922 linearly by controlling the prismatic joint 944a, a motor for rotationally translating the position of the implant in the "R" plane via a rotational mechanism 944b, and a further rotation motor configured to control rotational movement of the insertion module 930 in the rotation plane "Y" via a first rotatable portion/rotational gear 946 to adjust an angle of insertion.

The insertion module 930 comprises a second rotatable portion 948. The second rotatable portion 948 is configured to rotate the insertion module 930 in a pitch plane "P" to change the angular direction of implantation, as shown in Figure 9G. The second rotatable portion 948 is configured to rotate the insertion module 930 in a pitch plane "P" perpendicular to the yaw plane "Y" to change the angular direction of implantation.

The yaw and/or the pitch movement may be facilitated by a rotary base driven by a four-start worm gear, for example. The yaw and/or pitch movement may be controlled by a worm gear, or by a set of spur gears, for example. Two motors of the insertion module 930 may be used in some examples, one to actuate pitch movement and one to actuate yaw movement. Therefore, by adjusting the pitch and/or the yaw, the appropriate angle of insertion of the implant 200 may be achieved.

It will be appreciated in some examples that, for example, the mechanism to control the yaw movement in the "Y" plane as shown may be part of the insertion module 930 rather than the positioning module 920; the mechanism to control the pitch movement in the "P" plane as shown may be part of the positioning module 920 rather than the insertion module 930; in some examples the mechanism(s) to control both the pitch and the yaw movement may be part of the positioning module 920, or both may be part of the insertion module 930. Other variations on the particular locations of the degree of freedom movement mechanisms may also be envisaged.

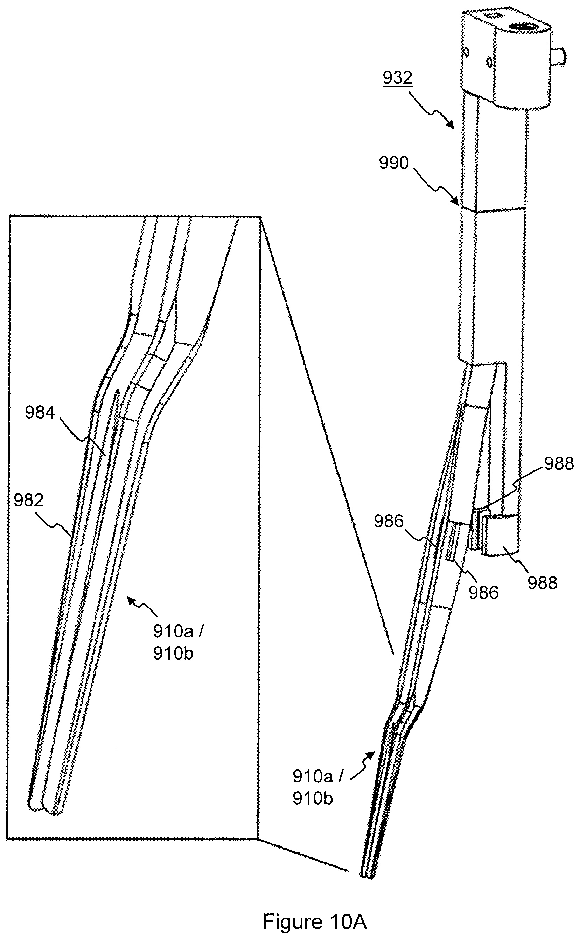

The insertion module 930 comprises an implant holder movement portion 932 configured to translate an implant holder 910 held in the insertion module 930 along a direction of implantation B as shown in Figures 9I and 9J. It can be seen from Figures 9I and 9J that the implant holder 910 may be comprised of a pair of implant supports 910a, 910b which may be linearly translated independently to control the translation movement of the supported implant. A gear system 950 may be used to control movement of the implant holder movement portion 932, and Figures 9H, 9I and 9J illustrate that different configurations of gears in the gear system 950 may be used to control movement of the implant holder 910 in the rotational direction J, and in the translational direction B (as shown, in some examples, two parts of the implant holder 910 may support the implant and may be translated independently of each other). One such example implant support is shown in Figure 10A.

The implant holder movement portion 932 allows for linear movement of the implant 200 along the second linear direction B, which may be controlled by two prismatic joints (e.g., lead screws) housed in the core/housing 934 of the insertion module 930. The prismatic joints may mount carriages to control the progression of the implant 200. The core/housing 934 may include two motors, for example which are operated using a movement mechanism 950 as shown, each motor being in charge of actuating the respective prismatic joints and advance/retract the implant 200 along the implantation "B" axis direction. This arrangement of elements is more accurate and allows better control compared to friction-based systems used in implant insertion because the contact point of the instrument with the implant may be closer to the point of insertion. The arrangement of two prismatic joints allows for the insertion of both straight and pre-curved implants. The use of prismatic joints (lead screws) to control movement of the implant holder 910 rather than friction-based systems (e.g. rollers), helps to improve grip contact with the implant to improve accuracy and control of insertion yielding a more consistent performance.

The insertion module 930 comprises an implant holder movement portion 932 configured to rotate the implant holder 910 about a first axis of rotation "J" along the direction of implantation B, as shown in Figure 9H. In this way the rotational orientation of the implant 200 may be adjusted for implantation. The implant holder movement portion 932 may be configured to allow roll to rotate the implant holder 910 in the "J" plane. This movement may be controlled by a set of spur gears 950 as illustrated. These may be integrated into the core/housing 934 of the insertion module 930. An additional motor may be mounted outside the core/housing 934 to drive the spur gear responsible for the roll movement.

In the examples illustrated, the arrangement of movement mechanisms as shown provides a compact surgical instrument with reliable implant positioning. As noted above, in examples of the instrument 900 being used to manipulate a surgical tool rather than an implantation device, the arrangement of movement mechanisms as shown provides a compact surgical instrument with reliable held tool positioning and movement.

The connection between the positioning module 920 and insertion module 930 may form a point at which the axes of degrees of freedom intersect. Position controlled by the positioning module 920 may be decoupled from pose controlled at the connection between the positioning module 920 and insertion module 930, and may also provide a remote centre of motion. The connection between the positioning module 920 and insertion module 930 disclosed herein incorporates rotation of the insertion module 930 in the pitch "P" and yaw "Y" directions, which is not known in the art.

By facilitating movement of the insertion module 930 in a polar workspace via linear movement along "A", pitch movement in the plane "P", and yaw movement in the plant "Y", instead of a cartesian coordinate system workspace, this allows the system 900 to be more compact and stable. It is also mechanically simpler, requires fewer components and is more efficient than other systems in the art. A cartesian system requires higher tolerances since the x, y, and z rails need to be precisely 90° (orthogonal) to each other. The polar system proposed herein also allows for greater freedom of design of the insertion module 930 since there is no interference with structural members. The arrangement of the positioning module 920 supporting and configured to move the insertion module 930 maximises the field of view under the microscope in the region of surgery, and makes it more intuitive for the user to understand what is happening. The modular arrangement also facilitates screwing of the instrument 900 to the patient's skull since the base 922 of the positioning model 920 may be connected to the patient first, and the insertion module 930 may be then connected to the positioning module 920.

In examples making use of a prismatic joint 944a (or other linear movement mechanism) to facilitate the linear movement, the joint can be supported on a stabilisation table to help eliminate error in the z-axis of the insertion module 930 due to deflection. The modular approach and joint type (prismatic joint for linear travel) described herein also facilitates reducing the instrument weight by using less stiff materials and still providing for stable implant insertion.

This arrangement of elements allows for rotation and insertion of the implant while still providing good visualisation to the surgeon at the surgery location on the body. As noted above, in examples of the instrument 900 being used to manipulate a surgical tool rather than an implantation device, the arrangement of elements allows for movement of the held surgical tool while still providing good visualisation to the operator at the surgery location on the body.

The implant holder 910 may be detachably connected to the implant holder movement portion 932, as shown in Figure 10A, which allows for the implant holder 910 to be removed and replaced or cleaned before re-mounting (or for replacement with a differently shaped and/or dimensioned implant holder 910), without necessarily requiring cleaning of the entire instrument 900 or insertion module 930 of the instrument 900.

The insertion module 930 may comprise a housing 934 as shown, configured to at least partially house the implant holder movement portion 932. The housing 934 may, as shown, comprise a body 936 and a plurality of arms 938 extended from the body 936. The arms 938 are configured to attach to the insertion module support 942.

The insertion module support 942 may comprise a semi-annular (e.g. C-shaped) or annular ring in some examples, configured to connect to the plurality of arms 938 and support the insertion module 930. By way of the arms 938 which are used to attach the insertion module 930 to the insertion module support 942 of the positioning module 920, the spaces between the arms 938 form a "cut-out" section which advantageously allows the field of view of a person or a microscope in the region of surgery to be improved.

The positioning module 920 may be detachably connected to the insertion module 930. Optionally, the positioning module 920 may be detachably connected at the points where the annular / semi-annular ring 942 is connected to each of the plurality of arms 938. The annular / semi-annular ring 942 and/or the insertion module 930 may be detached from the positioning module 920 to facilitate loading of an implant into the implant holder 910 held in the insertion module 930 in use.

The positioning module 920 may further comprise an external support portion 920a which is configured to attach to an external supporting element (not shown). The surgical instrument 900 may further comprise the external supporting element in some examples, e.g. an arm to attach to a patient bed.

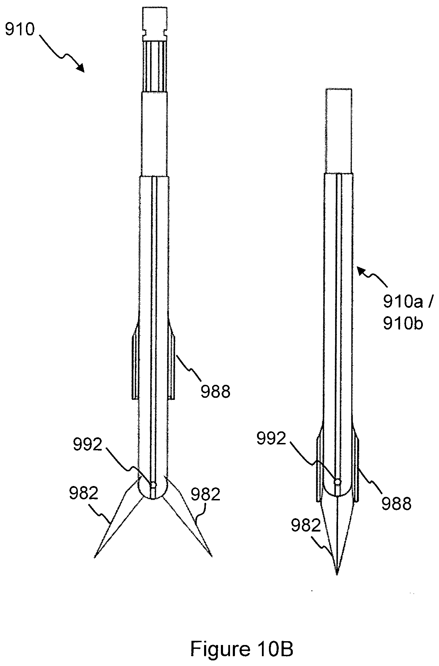

The implant holder 910 may comprise an implant contact portion 982 as shown in Figures 10A and 10B. Figure 10A shows one half of an implant holder 910, wherein one implant support 910a, 910b is shown which may be used in conjunction with a similar symmetrical second implant support 910a, 910b to form an overall implant holder 910 as illustrated in Figures 9A-9J. The implant contact portion 982 may comprise a channel 984 (which may also be called a groove or carved section) in the implant holder 910 (herein in the implant support 910a, 910b) which is configured to adapt to the shape of the implant and improve the contact area with the implant. Figure 10B shows a different arrangement of implant holder 910 with a pair of jaws 982.

The implant contact portion 982 of either example may comprise a deformable material configured to conform to the shape of an implant 200 held in the implant holder 910. The implant contact portion 982 may comprise a patterned surface configured to grip an implant 200 held in the implant holder 910. The implant contact portion 982 may be made of a soft material to conform around the implant parts, such as a soft polymer, deformable metal, shape memory polymer, or other material. A pattern on the implant contact portion 982 surface may increase the friction between the implant contact portion and the implant to facilitate secure gripping. Such an arrangement may help to securely grip the implant while reducing the risk of damage to the implant due to gripping. The implant device may comprise a clamping portion configured to hold an implant by clamping.

The positioning module 920 and/or the insertion module 930 may comprise a tracking feature, such as an optical marker, to facilitate autonomous control of the position and/or pose of the implant with respect to the patient's anatomy opening before and during the implant insertion.

The implant holder 910 may be removably connected to the implant holder movement portion 932 to facilitate connection and detachment of the implant holder 910 from the implant holder movement portion 932. In this way the implant holder 910 may be removed and replaced/cleaned easily and the remainder of the instrument 900 may be reused. The insertion module 930 may comprise a lead screw actuated by a motor, and a carriage may be attached to the lead screw. The carriage moves linearly; and the implant holder 910 may be attached to the carriage to facilitate linear movement of the implant holder 910.

The instrument 900 may may include a connection feature, such as a magnetic, snap fit, press fit or combination of these, to allow for the implant holder 910 to be connected to the insertion module 930 and held in place. For example, the implant holder 910 may be plugged into an extension from carriages driven by the two prismatic joints from the insertion module 930. The feature of the implant holder 910 being removable allows the sterilisation and reuse of the positioning and insertion modules. The implant holder may be the only single-use component in the instrument 900, as it may be removed and cleaned/replaced as facilitated by the arrangement of modules in the modular instrument 900. In some examples, the insertion module 930 may be detached from the insertion module support 942 which may be beneficial once the implant is attached to the device, to allow a surgeon to continue an implant procedure manually or to carry it manually from the beginning if the surgeon prefers it or considers it necessary. In some examples, the insertion module 930 may be detached from the positioning module at another connection point. For example, the implant holder movement portion 932 and implant holder 910 may be detached from the housing 934 following disconnection of the large gear shown as part of the gear system 950 and then pushing implant holder movement portion 932 up as illustrated. As another example, the second rotatable portion 948, implant holder movement portion 932 and implant holder 910 may be detached from the insertion module support 942 after disconnecting the corresponding support element /arms 938 from the insertion module support 942.

In examples where a surgeon wishes to operate the instrument 900 manually, there may be an ergonomic shape to the instrument 900, or there may be an ergonomic casing configured to attach to the instrument, or to a part of the instrument (e.g. to the housing 934 containing the implant holder movement portion 932, implant holder 910 and gear system 950) to allow the user to manipulate the instrument. The surgeon may be able to manipulate the instrument manually in situ or remotely by way of one or more controls connected to the instrument 900, for example, one or more joysticks, directional pads trackballs, foot pedals, buttons, or other user input devices.

The implant holder 910 may be removably connected to the implant holder movement portion 932 to facilitate connection and detachment of the implant holder 910 from the implant holder movement portion 932. As best seen in Figure 10A, the implant holder 910 (here, the implant support 910a, 910b) may comprise at least one engagement recess 986 (as shown in the example of Figure 10A there is an opposite pair of engagement recesses 986). The implant holder movement portion 932 may comprise at least one wing (e.g. an opposite pair of wings 988) corresponding to the at least one engagement recess 986. Each wing 988 is configured to engage with an engagement recess 986 of an implant holder 910 (or implant support 910a, 910b) connected thereto, to secure the implant holder 910 / implant support 910a, 910b to the implant holder movement portion 932. The wings 988 may be shaped and/or dimensioned to facilitate implant holder 910 clamping. In other words, the engagement portions 986 may be engaged and held by a tightening feature (the wings 988 or a tab or protrusion of each wing 988 which can be located within the corresponding engagement portion 986). These features may assist the user with the clamping procedure of clamping an implant in the implant holder 910. The one or more wings 988 may be movable wings and there may be a mechanism to tighten and release the wings 988 to clamp and release an implant. The wings 988 may be controlled, for example, whether or not the implant holder 910 is, connected to the insertion module 930.

The implant holder 910 may be one of plural available implant holders each having a differently dimensioned channel 984 to allow for implantation of implants of different dimensions. That is, different implant holders 910 may be available and advantageously attachable for use with different implants.

Figure 10B illustrates an alternative implant holder 910 to that of Figure 10A. In this example, the implant holder 910 comprises a point 992 around which two implant contact portions 982 of the implant holder 910 can pivot. These implant contact portions 982 can be secured/tighten with the help of the movable wings 988 in some examples as shown, which in this example slide over a portion of each implant contact portion 982 to hold it closed. The wings 982 may be of the implant holder 910 or of the implant holder movement portion 932.

Looking back to the example of Figure 10A, in some examples there may be a drape which is connected at the implant holder 910, for example at a drape connection point 990, and which may cover the whole robotic system 900 (except for the protruding portion of the implant holder 910) to keep the instrument 900 separate from the implant holder 910 and patient in use. The drape may be attached to the groove 986 in the implant holder 910 in some examples.

The drape connection point 990 may be used with a custom drape having an internal connecting portion configured to attach to the non-sterile part of the instrument 900, and an external portion configured to attach to the sterile portion of the instrument 900. The drape connection point 990 may comprise a sterilisable connecting portion that would act as interface between the non-sterile draped part of the instrument 900 and the sterile and disposable part of the instrument 900 (i.e., part of the implant holder movement portion 932 and the implant holder 910). In this example the drape can be a simple "plastic bag" which would reduce costs.

It will be appreciated that in examples where the instrument 900 is used to hold a tool rather than an implant, the portion 910 may be a tool holder with features to assist in advantageously hold a surgical tool for movement by the instrument 900.

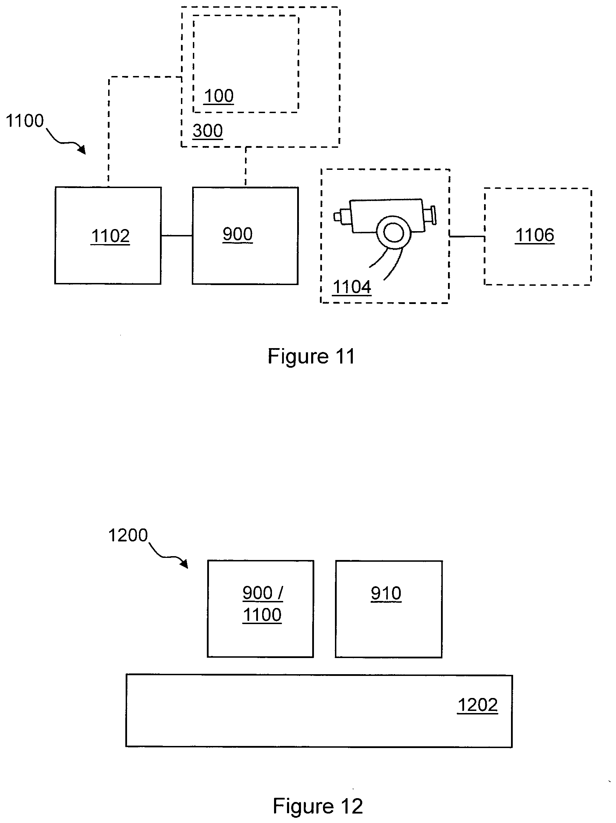

Figure 11 shows a surgical system 1100 comprising a surgical instrument 900 and a controller 1102. The controller 1102 is configured to operate the movement of one or more of the translation portion 944, the first rotatable portion 946, and the implant holder movement portion 932 of the surgical instrument 900 by operating a respective movement mechanism of the translation portion 944, the first rotatable portion 946, and the implant holder movement portion 932. The controller 1102 may be configured to communicate with one or more actuation units (e.g. motors) of the instrument 900 as discussed in relation to Figures 9A-9J. The controller 1102 may comprise a controller for each actuation unit and a main board responsible for communicating with an external computing apparatus such as a PC. The controller 1102 may be configured to generate the positioning and insertion motion commands with which to control the instrument 900. The motion commands may vary depending on the implant loaded in the insertion unit of the instrument 900.

The surgical system 1100 may also comprises an apparatus 100 according to examples disclosed herein such as the apparatus 100 discussed in relation to Figure 1. As illustrated the apparatus 100 is part of a system as disclosed in relation to Figure 3, and is connected to the instrument 900. In other examples the apparatus 100 and/or system 300 may be part of the instrument 900.

The controller 1102 is shown in connection with the system 300. For example, the apparatus 100 may receive feedback data from the controller 1102 indicative of the impedances being measured and may use that feedback data to determine one or more parameters relating to the insertion of an implant, such as distances, implant orientation, etc as discussed above. The apparatus 100 may provide control signalling to the controller 1102 to cause the controller 1102 to operate the instrument 900 according to the received feedback, for autonomous implant insertion. Thus, the surgical system 1100 may further comprise any apparatus 100 disclosed herein, or any system 300 disclosed herein; wherein the apparatus 100 or system 300 is configured to output the determined distance between the electrode contact surface and a wall of the electrically conductive volume to the controller, to cause the controller 1102 to operate the movement of the one or more of the translation portion, the rotatable portion, and the implant holder movement portion of the surgical instrument 900 to perform implantation of the implant in the electrically conductive volume of the patient. The apparatus 100 or system 300 of the surgical system 1100 may be configured to: determine, in dependence on the determined distance between the electrode contact surface and a wall of the electrically conductive volume, one or more operation controls to provide to the controller 1102 to cause the controller 1102 to operate the movement of the one or more of the translation portion, the rotatable portion, and the implant holder movement portion of the surgical instrument 900; and output the one or more operation controls to the controller 1102.

It will be appreciated that the connections shown in Figure 11 are schematic and that the various elements may be connected in different ways and achieve the functionality disclosed.

The surgical system 1100 may further comprise a microscope 1104 configured to capture images of the location of insertion of an implant into an electrically conductive volume in a patient. The surgical system 1100 may further comprise a display screen 1106 apparatus configured to receive image signalling from the microscope 1104 and display the captured images of the location of insertion of the implant. The images captured can be augmented with overlays and relevant information to provide better intraoperative feedback to the surgeon.

Figure 12 shows a surgical instrument system 1200 comprising a surgical instrument 900, surgical system 1100, or implant holder 910, and a loading rig 1202 according to examples disclosed herein. The loading rig 1202 may be configured to support an implant holder 910 to facilitate the clamping / holding of an implant in the implant holder 910. The loading rig 1202 may be configured to support at least a part of the surgical instrument 900 or system 1100 to facilitate loading and unloading of an implant holder 910 in and from the implant module 930. The loading rig 1202 may be configured to hold the implant holder 910 to allow a user to insert an implant and manually clamp the implant in the implant holder 910.

Use of the surgical instrument 900 or system 1100 for robotically assistant implant insertion may be according to a method comprising the following steps (it will be appreciated not all steps are essential and the order could be altered):
a. detaching the insertion module support 942 and insertion module 930 from the rotary base 922, and resting them over the loading rig 1202;
b. advancing the insertion module 930 prismatic joints until the carriages are visible through the easy access portion;
c. connecting the drape to the drape connection portion 990, and in some cases, to a sterilisable portion as described above;
d. securing the sterile and disposable portion of the implant holder movement portion 932 to the drape connection portion 990 and connecting, without securing, the sterile and disposable implant holders to the disposable portion of the implant holder movement portion 932;
e. loading and securing the implant into the clamps of the sterile and disposable implant holder 910;
f. determining, using pre-operative/intraoperative data, the optimal insertion angle, which can be displayed in a headset, monitor or integrated into the operating microscope;
g. calibrating the insertion module;
h. adjusting the position of the insertion module tip:
   i. assisted, by actuating the movable parts of the controller. The movable parts map to all the joints in the base 922 and 944, as well as the rotation drive by the gear system 950; or
   ii. autonomously using a tracking system of the instrument 900;
i. confirming the position;
j. adjusting the orientation of the insertion module tip:
   i. assisted, by actuating the movable parts of the controller. The movable parts map to all the joints in the insertion module support 942 and rotation in the insertion module 930 (that is, all available degrees of freedom may be involved in this step); or
   ii. autonomously using the tracking system;
k. confirming the orientation;
l. placing the implant into the anatomy cavity:
   i. assisted, by actuating the movable parts of the instrument 900, for example using user input devices to control motors of the instrument 900.
   ii. autonomously, using a closed-loop or open-loop feedback system based on the impedance as discussed above in relation to Figures 1 to 8;
m. monitoring the positioning of the implant;
n. determining, using the feedback system, that the implant is in a first target position;
o. determining, using the feedback system, that the implant has reached a second target position; and
p. monitoring impedance values after insertion.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, and characteristics described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed. The scope of protection is defined by the appended claims.

## Claims

1. An apparatus for controlling insertion of an implant into an electrically conductive volume in a patient, the implant comprising a plurality of electrodes arranged on an electrode contact surface of the implant, the apparatus comprising a memory configured to store computer-readable instructions; and a processor configured to execute the computer-readable instructions which, when executed, cause the apparatus to:
transmit, to a pulse generator, at least a first electrode operation signal configured to generate at least a first current pulse on at least a first pair of electrodes of the implant;
receive a first impedance signal from a first measurement pair of electrodes, the first impedance signal indicative of a first impedance due to interaction of the at least a first current pulse with the electrically conductive volume;
receive a second impedance signal from a second measurement pair of electrodes, the second impedance signal indicative of a second impedance due to interaction of the at least a first current pulse with the electrically conductive volume, wherein the first measurement pair of electrodes and the second measurement pair of electrodes are different;
determine, in dependence on the first impedance signal and the second impedance signal, and on a predetermined mapping relationship, a distance between the electrode contact surface and a wall of the electrically conductive volume; and
output the determined distance between the electrode contact surface and a wall of the electrically conductive volume.

2. The apparatus of claim 1, wherein:
the at least a first pair of electrodes is a single pair of electrodes; and
the at least a first electrode operation signal is a single electrode operation signal.

3. The apparatus of any preceding claim, wherein the apparatus is configured to receive the first impedance signal and the second impedance signal simultaneously.

4. The apparatus of any preceding claim, wherein the first impedance signal is indicative of a tetrapolar impedance, received based on a voltage difference between the first measurement pair of electrodes, the first measurement pair of electrodes comprising:
a distal first measurement electrode positioned at a distal end of the first pair of measurement electrodes, and
a proximal first measurement electrode positioned at a proximal end of the first pair of measurement electrodes;
so the at least a first pair of electrodes are located between the distal and proximal first measurement electrodes.

5. The apparatus of any preceding claim, wherein the second impedance signal is indicative of a bipolar impedance, received based on a voltage difference between the second pair of electrodes.

6. The apparatus of any preceding claim, configured to:
transmit, to the pulse generator, a first further electrode operation signal configured to generate a first further current pulse on a further pair of electrodes of the implant, wherein the further pair of electrodes comprises at least one different electrode to the at least a first pair of electrodes so the further pair of electrodes are separated by a different amount than the at least a first pair of electrodes;
receive a first further impedance signal indicative of a first further impedance due to interaction of the first further current pulse with the electrically conductive volume; and
determine the distance between the electrode contact surface and a wall of the electrically conductive volume further in dependence on the first further impedance signal.

7. The apparatus of any preceding claim, further configured to
transmit, to the pulse generator, a third electrode operation signal configured to generate a third current pulse on a third pair of electrodes of the implant;
receive a third impedance signal indicative of a third impedance due to interaction of the third current pulse with the electrically conductive volume, received based on a voltage difference between a third measurement pair of electrodes, the third measurement pair of electrodes comprising:
a common third electrode of both the third pair of electrodes and the third measurement pair of electrodes; and
a further third electrode of the electrode array, the further third electrode different from the third pair of electrodes.

8. The apparatus of any preceding claim, further configured to:
transmit, to the pulse generator, a fourth electrode operation signal configured to generate a fourth current pulse on a fourth pair of electrodes, the fourth pair of electrodes comprising a reference electrode external to the electrodes of the implant, and a fourth electrode of the electrodes of the implant;
receive a fourth impedance signal indicative of a fourth impedance due to interaction of the fourth current pulse with the electrically conductive volume, received based on a voltage difference between a fourth pair of electrodes.

9. The apparatus of any preceding claim, further configured to:
transmit one or more of:
a plurality of first electrode operation signals each at a different frequency, and
a plurality of second electrode operation signals each at a different frequency; and
determine the distance between the electrode contact surface and a wall of the electrically conductive volume in dependence on one or more of:
a plurality of first impedance signals respectively received due to the plurality of first electrode operation signals, and
a plurality of second impedance signals respectively received due to the plurality of second electrode operation signals.

10. The apparatus of any preceding claim, wherein the predetermined mapping relationship is determined using a machine learning model trained using:
first impedance signal training data,
second impedance signal training data, and
training data distances between the electrode contact surface and a wall of the electrically conductive volume corresponding to the first and second impedance signal training data.

11. The apparatus of any preceding claim, configured to output the determined distance between the electrode contact surface and a wall of the electrically conductive volume to one or more of:
an output device configured to provide an indication of the output to a user; and
a surgical instrument controllable using the output to perform insertion of the implant into an electrically conductive volume in a patient.

12. A system for controlling insertion of an implant into an electrically conductive volume in a patient, the system comprising:
the apparatus of any of claims 1 to 11; and
the pulse generator.

13. The system of claim 12, further comprising:
a front-end amplifier configured to receive the first impedance signal and the second impedance signal simultaneously.

14. The system of claim 12 or claim 13, further comprising:
a multiplexer configured to select at least the first pair of electrodes and the second pair of electrodes from the plurality of electrodes of the implant.

15. A surgical instrument for implant insertion, the surgical instrument comprising:
a positioning module: and
an insertion module;
the positioning module comprising:
a patient-attachable portion configured to attach to a patient; and
an insertion module mechanism comprising:
an insertion module support configured to support the insertion module;
a translation portion configured to translate the supported insertion module along a first linear direction to change an entry position of implantation; and configured to rotationally translate the supported insertion module in a first plane of rotation to change the entry position of implantation; and
a rotatable portion configured to rotate the supported insertion module in a yaw plane to change the angular direction of implantation;
the insertion module comprising:
a rotatable portion configured to rotate the insertion module in a pitch plane to change the angular direction of implantation; the pitch plane perpendicular to the yaw plane; and
an implant holder movement portion configured to translate an implant holder held in the insertion module along a second linear direction along a direction of implantation and configured to rotate the implant holder about a first axis of rotation along the direction of implantation.

16. The surgical instrument of claim 15, wherein the insertion module comprises a housing configured to at least partially house the implant holder movement portion,
the housing comprising a body and a plurality of arms extended from the body, the arms configured to attach to the insertion module support.

17. The surgical instrument of claim 16, wherein the insertion module support comprises an annular ring or partial ring configured to connect to the plurality of arms and support the insertion module.

18. The surgical instrument of any of claims 15 to 17, wherein the positioning module is detachably connected to the insertion module.

19. The surgical instrument of any of claims 15 to 18, wherein the positioning module further comprising an external support portion configured to attach to an external supporting element.

20. The surgical instrument of any of claims 15 to 19, further comprising an implant holder configured to releasably hold an implant for insertion into a volume.

21. The surgical instrument of claim 20, wherein the implant holder comprises an implant contact portion, the implant contact portion comprising one or more of:
a deformable material configured to conform to the shape of an implant held in the implant holder; and
a patterned surface configured to grip an implant held in the implant holder.
a groove, channel, or carved section configured to adapt to the shape of an implant located therein.

22. The surgical instrument of claim 20 or claim 21, wherein:
the implant holder is removably connected to the implant holder movement portion to facilitate connection and detachment of the implant holder from the implant holder movement portion;
the implant holder comprises at least one engagement recess; and
the implant holder movement portion comprises at least one wing corresponding to the at least one engagement recess, the at least one wing configured to engage with the at least one engagement recess of an implant holder connected thereto to secure the implant holder to the implant holder movement portion.

23. A surgical system comprising:
the surgical instrument of any of claims 15 to 22; and
a controller;
the controller configured to operate the movement of one or more of the translation portion, the rotatable portion, and the implant holder movement portion of the surgical instrument by operating a respective movement mechanism of the translation portion, the rotatable portion, and the implant holder movement portion.

24. The surgical system of claim 23, further comprising:
the apparatus of any of claims 1 to 11; or
the system of any of claims 12 to 14;
the apparatus or system configured to output the determined distance between the electrode contact surface and a wall of the electrically conductive volume to the controller, to cause the controller to operate the movement of the one or more of the translation portion, the rotatable portion, and the implant holder movement portion of the surgical instrument to perform implantation of the implant in the electrically conductive volume of the patient.

25. A surgical instrument system comprising:
the surgical instrument of any of claims 15 to 22 or the surgical system of claim 23 or 24; and
a rig configured to support at least a part of the surgical instrument to facilitate loading and unloading of an implant in and from the implant holder.
